# EUROPEAN PATENT SPECIFICATION

(11) **EP 1 866 226 B1**
(45) Date of publication and mention of the grant of the patent: **25.08.2010**
(21) Application number: 06723316.3
(22) Date of filing: 09.03.2006
(51) Int. Cl.: B65H 20/32

(54) **PROCESS AND EQUIPMENT FOR MANUFACTURING THREE DIMENSIONALLY SHAPED OR BELTED ARTICLES, SUCH AS GARMENTS OR ABSORBENT ARTICLES**
VERFAHREN UND EINRICHTUNG ZUR HERSTELLUNG VON DREIDIMENSIONAL GEFORMTEN ODER MIT GÜRTEL AUSGESTATTETEN ARTIKELN, WIE ZUM BEISPIEL KLEIDUNGSSTÜCKEN ODER ABSORBIERENDEN ARTIKELN
PROCEDE ET EQUIPEMENT DESTINES A LA FABRICATION D'ARTICLES DE FORME TRIDIMENSIONNELLE OU A CEINTURE, TELS QUE DES VETEMENTS OU DES ARTICLES ABSORBANTS

(30) Priority: 29.03.2005 WO PCT/IB2005/000845
(43) Date of publication of application: 19.12.2007
(73) Proprietor: Concepts For Success, 53881 Euskirchen Stotzheim (DE)
(72) Inventor: SCHMITZ, Christoph, 53881 Euskirchen Stotzheim (DE)
(74) Representative: Plischke, Manfred
(86) International application number: PCT/EP2006/002165
(87) International publication number: WO 2006/102973

(56) References cited:
- DE-A1- 2 447 656
- US-A- 5 693 165
- US-A- 5 979 308

## Description

### Field of the invention

The present invention relates to a method and equipment for the manufacturing of articles from web materials, such as textiles, non-wovens, films, or composites made there from. The articles have a three dimensional shape and/or comprise a belt or a hoop struchue. In particular, it can be applied to the production of wearing apparel or garments, or of absorbent hygiene articles, such as disposable diapers.

### Background

The industrial manufacturing of articles such as garments having a three-dimensional (3D) shape or comprising a closed structure, such as a belt or a hoop, conventionally employs little automation, e.g. manual sewing of the components. Automated methods are rarely used, and are typically limited by complex designs and/or low production speeds. Such sewn articles are disclosed in EP-A-0988803, relating to a panty girdle, or US-A-2004/0098784, relating to a net pant, or WO-A-01/061093, relating to boxer shorts, however the manufacturing is not on production lines employing a continuous process.

In GB-A-2013326, the use of automated sewing machines in combination with a circular guide rail and an annular carrier to transport a pair of stocking material through several stations, such as seaming stations, is disclosed. Whilst this complex and heavy mechanical set up allows automated production, it is severely limited in production speed. US-B-04803935 discloses a method and an apparatus for forming belt loops and transferring the formed loop to a sewing station where the loop may be stitched to the waistband of a pair of trousers.

In US 5979308 a high speed embossing machine is disclosed where a continuously supplied embossing film is intermittently formed into loops so as to allow embossing at no or reduced relative speed between the embossing film and the material which is to be embossed.

In WO-A-96/07376 absorbent pads are described, which are formed into a three dimensional shape by a thermoforming process. JP01284284A2 discloses a machine for continuous sewing of an annular resilient belt-form material. The machine inserts or sews a tubular elastic belt shape body, e.g. rubber ring, to tubular cloth, e.g. trousers or a skirt, continuously. The machine sews the cloth by guiding the rubber ring by a guide device having a guide plate, bending ends of tubular cloth to make a bent part, and placing endless rubber ring in the space made by the bent part and retreats to the open end side of the bent part. In US application US-A-2002/0084017, a process is described for the manufacturing of a 3D article, wherein waist elastic, an insert and a 3D skirt or trunk are combined by using an expandable/ retractable fixture, which is run through a process loop such as a loop conveyor system. This process and equipment however, is complex in design, and whilst the process may run automated, there are also severe limitations in the production speed.

Hence there remains the desire for manufacturing of complex articles at highly efficient production equipment.

### Summary

A solution to this problem is provided by the present invention, which is a method for manufacturing shaped articles from one ore more web materials on a manufacturing equipment, wherein the articles form a closed hoop structure or a three-dimensional structure and have an inner and an outer surface, and are formed by connecting one or more regions of one or more web pieces of one or more web materials. The web materials are essentially flat by having a length dimension (x- direction) and a width dimension (y- direction) exceeding the thickness dimension (z-direction), thus having a first and a second surface along the x-y-direction. The web materials may form a sequence of web material pieces. The method is an essentially continuous manufacturing process, comprising at least one web treatment step acting on the inner and outer surfaces simultaneously by a web treatment unit comprising a web treatment head and a counteracting web treatment tool. The manufacturing equipment comprises a web path splitter means for parallel treatment of at least two web pieces in at least two web treatment sections, each of these sections comprising a treatment head of the web treatment unit and at least a first and a second web support means for temporarily holding and moving the web material pieces. Thereby, the second web support means is positioned z-directionally offset relative to the first web support means, thus forming a gap between the first and second web support means, wherein the gap distance between the first and the second web support means can be varied. The method comprises steps of
(a) feeding one or more pieces of web material via the web path splitter means to the web treatment sections;
(b) temporarily attaching a first region of a piece of a web material to the first web support means and a second region of the same or a different piece of a web material to the second web support means, such that the first and the second regions are positioned in the gap between the first and the second web support means;
(c) increasing the z-directional distance of the first and second web support means whilst the first and second regions of the web material(s) remain at least partly affixed to the respective web support means, so as to increase the gap distance between the web support means and the regions of the web material piece(s) affixed thereto,
(d) positioning the web treatment head in the gap to contact the surfaces of the web material regions oriented towards the web treatment head;
(e) positioning the web treatment head and the web treatment tool relative to each other and to the web material so as to allow them to interact each with one of the opposite surfaces of the web material(s);
(f) treating the first and the second web regions jointly by cooperatively operating the web treatment head and the web treatment tool of the treatment unit, thereby forming the structure of the article;
(g) removing the web treatment head from the gap and/or the article from the head;
(h) optionally further treating the article;
(i) removing the article from the web handling equipment.

The web materials may be made of films, textile, woven, or non-woven webs, or composites thereof. The first and second regions may belong to different webs; which may be of the same or different material type, orientation, or which may have undergone different pre-treatments. The webs material(s) may be pre-shaped webs, e.g. forming a closed structure like a belt or a hoop. The method may further include the use of other materials, such as pre-assembled pieces, bulk material, or fluids, and several process steps may be executed on one piece of web material. A preferred treatment step is the connecting of two regions, but further treatment steps may be included, such as cutting, pressing, or mechanically activating of webs.

The method is particularly suitable for the manufacture of shaped articles as garments for being worn on the lower torso, preferably as disposable garments or disposable absorbent articles. The articles may further comprise any or all of the following elements
(i) a hoop structure defining the waist opening of the article;
(ii) two hoop structures defining the leg openings of the article;
(iii) a centre piece connecting the front and rear regions through the crotch region of a wearer during use;
(iv) side panel materials positioned in the hip region of the wearer during use;
(v) the centre piece being connected to the leg hoops, preferably by a curved connecting line;
(vi) the side panels being connected to front and back parts of the centre piece, and either to the leg hoop, or to the waist hoop, or to both.

A particular embodiment of the invention relates to a method for handling a web material, which is an essentially continuous web or an essentially continuous sequence of pieces of an essentially continuous web, on a web handling equipment. The web handling equipment has an overall web path connecting a web supply means with a process section end point, whereby the web material comprises at least a first and second section being connected and spaced apart with the first section being oriented along the overall web path more towards the process section end point than the second section, wherein the method comprises the steps of
a) providing the web material on the web supply means;
b) moving the web material from the web supply means towards the process end section along the overall web path at an overall web path speed |v₀| relative to the frame of the web handling equipment;
c) providing a web path splitting means positioned along the overall web path and comprising at least a first and a second web handling section,
each of these web handling sections comprising a section frame, and at least one web support means connected to the section frame, having a surface which is movable relative to the section frame;
d) splitting the web path on the web path splitting means into at least a first and a second web sub-path, each running through one of the web handling sections; and transferring the web material along the web sub-paths to the web handling sections;
e) handling the web material in each of the web handling sections by affixing at least the first section of the web material to the surface of a web support means in the initial contact region of the web support means, without affixing the second section of the web material to the web support means surface; and by changing the speed of the surfaces of the web support means, while having at least the first section of the web material remaining affixed thereto, thereby changing the relative speed of the first section of the web material to a second section of the web material,
f) thereby transferring at least parts of the web material out of the initial contact regions of the web support means into an operating region of the web support means or of a further web support means, thereby forming a cross-directional fold in the web material;
g) performing at least a further web handling or treatment steps on the web material, preferably at least a step connecting two regions of one or two webs to each other;
h) moving the web material from the web handling section;
i) providing the web handling section for repeated executions of the web handling steps d) to h). The present invention also relates to an apparatus for manufacturing shaped articles from one ore more web materials, the apparatus comprising
(i) a web path splitting means comprising at least two web treatment sections; preferably a rotatably mounted drum;
(ii) a web treatment unit, comprising at least two web treatment elements in the form of a web treatment head and a web treatment tool, which interact at least for the treatment of the web;
(iii) the web treatment sections comprising at least a first and a second web support means comprising moveable surfaces to which the web materials can be temporarily affixed, and an element of the web treatment unit, preferably a treatment head.

Therein, the web support means may comprise means to freely program the surface speed and direction, and the first and the second web support means may be arranged and comprise means to adjust their relative positioning, thereby forming a gap with a variable gap distance between them. The web treatment head and the first and second web support means may also be arranged and comprise means to adjust their relative positioning to allow positioning of the treatment head in the gap.

In a particular embodiment, the web path splitting means is a drum, which is rotatable around its longitudinal axis, preferably comprising four, more preferably six, and most preferably eight web treatment sections. However, even higher numbers of treatment sections per web path splitting means, such as 30 or 60, may be employed.

The apparatus may comprise further web support means, and the web support means may comprise any or all of endless transport belts, at least one drive roll, a freely programmable drive motor, preferably a servo motor, non-contact power and data transfer to the drive roll, or vacuum suction means.

The web treatment head of the apparatus may comprise any or all of a means for affixing web materials, for repositioning the web materials, for repositioning the treatment head be this rotatably or translatorily, for expanding or reducing certain dimensions of the treatment head; for treating the web material in cooperation with the treatment tool, whereby at least one of the treatment means may allow connecting web material(s). The web treatment head may further comprise a moveable surface, preferably in the form of a transport belt comprising vacuum suction means and a freely programmable drive motor and a non-contact power and data transfer. One treatment head may interact with one or more treatment tools, and one treatment tool may interact with one or more treatment heads. The treatment tool may be affixed to the web treatment station, and be stationary or repositionable relative to the treatment head; or it may be affixed to the manufacturing apparatus, and thusly consecutively interact with consecutive treatment heads, as these pass by. The treatment elements may further comprise rolls, anvils, spraying, or cutting units. The apparatus may further comprise sensing devices and control means such as for the detecting and correcting position of web materials.

The apparatus is particularly suitable for the manufacture of shaped articles as garments for being worn on the lower torso, preferably as disposable garments or disposable absorbent articles. The shaped articles may comprise any or all of the following elements
(i) a hoop structure defining the waist opening of the article;
(ii) two hoop structures defining the leg openings of the article;
(iii) a centre piece connecting the front and rear regions through the crotch region of a wearer during use;
(iv) side panel materials positioned in the hip region of the wearer during use;
(v) the centre piece being connected to the leg hoops, preferably by a curved connecting line;
(vi) the side panels being connected to front and back parts of the centre piece, and either to the leg hoop, or to the waist hoop, or to both.

### Brief description of the Figures

- Fig. 1 A - E: show examples of shaped articles in belt- or tube form, and simple pant designs;
- Fig. 2 A - C: show pant-like designs comprising belt structures;
- Fig. 3 A - B: show a 3D-shaped pant;
- Fig. 4 A - B: show shaped articles in the form of a pantyhose and a combination of an insert with a skirt like structure;
- Fig. 4 C - D: show a 3D cup shaped article;
- Fig. 5 A - D: schematically show fit lines of articles in the crotch region of a wearer;
- Fig. 6 A - D: show a shaped article comprising leg seal cuffs;
- Fig. 7 A - E: show shaped articles comprising a leg seal cuff with a leg hoop;
- Fig. 8 A - C: depict schematically the lay out for the equipment for an exemplary execution of the present invention;
- Fig. 9 A - E: schematically show exemplary arrangements of webs treated according to the present invention by a treatment unit;
- Fig. 10 A - O: depict individual process steps for a process according to the present invention;
- Fig. 11: shows a schematic set up for a treatment head;
- Fig. 12 A - C: show schematically details of a treatment head;
- Fig. 13 A - G: depict the process steps for creating side seams for pants;
- Fig. 14 A - B: depict schematically details of a treatment head;
- Fig. 15: depicts schematically the lay out for the equipment for an alternative exemplary execution of the present invention;
- Fig. 16 A - H: show schematically the set up to form belted pants;
- Fig. 17 A - D: show schematically the set up for forming a product comprising leg hoops;
- Fig. 18 A -B: schematically depict details of a forming head for forming a leg hoop;
- Fig. 19 A - C: schematically show process and equipment details for an optional process step (ear flipping);
- Fig. 20 A - C: depict schematically the use of a swivelling treatment head;
- Fig. 21: shows an exemplary process sequence chart.

The same numerals in various figures refer to corresponding elements.

### Detailed description

The present invention is concerned with a method of converting parts or pieces of a web material into shaped articles.

Generally, the term "web" relates to any material, which is essentially endless or continuous in one direction (generally denoted as "x-direction" or "machine direction" or MD). Webs are often, but not necessarily, stored, supplied, or used in roll form and thusly also sometimes denoted "roll goods". Whilst these are then not "endless" in the strict sense of the word, their extension in this x-direction is significantly larger than in any other direction. By combining consecutive rolls or other batches, ("splicing") such webs can be considered "endless" for all practical purposes. Webs may be transported in a "batch" form, such as when a roll thereof is shipped, or they may follow a "web path", such as when the webs are unwound from a roll.

Often, but not necessarily, webs have along their x-directional length an essentially uniform thickness (herein denoted as "z-direction"), and/or constant width (herein denoted as "y-direction" or cross-machine direction or CD). Webs may be of essentially uniform composition, they can be mixtures of materials, they can be composites of materials such as being layered (different materials arranged in a juxtaposed position in the z-direction) and/or can comprise stripes of different materials or materials having different or varying properties (i.e. arranged in a juxtaposed position in the y-direction).

Thus, such web materials have two opposed surfaces extending in the x-y-direction of the web, and being spaced apart in the z-direction by the thickness of the web material. Within the context of the present description, the surfaces remain the first and second surface of the material, even if the material is cut into pieces, or turned, or bent, or inverted or otherwise treated. Hence, when a piece of a web material is folded once, the surface of two regions of the web material will be in contact with each other. This contact can be directly, or indirectly, such as when a further material is interposed between the two surfaces, such as a further web material. Typical examples for webs are - without implying any limitation - plastic films or foils, textiles, non-wovens, nets, scrims, paper, or cartons.

When essentially endless continuous web materials are converted to form discrete articles, they will at some point in time be cut or otherwise separated into an essentially continuous sequence of such web materials. When such continuous sequence is moved, it will form a "sequence path", which may be considered as a web path. For example, if an essentially endless or continuous web, which is moved along a web path, is separated into pieces by repeatedly cutting off a certain length of the leading end of the web, the resulting sequence of cut pieces can still be considered a web material, following a web path, which is a continuation of the path which the continuous web followed before it was cut. As web materials in the form or parts or pieces follow the web path, they still have an orientation along this web path, which may be changed such as in case of rotating the web material or parts or pieces thereof. The sequence and the orientation along a web path distinguish web materials in the form of discrete parts or pieces from "sheets" for which neither a sequence nor an orientation along the web path can be defined. Thus, if the discrete parts or pieces of a web material are moved into a unit where they are stacked one upon the next to form a stack or staple, it would not be considered a web material, but rather a sheet, and the web path would end at the stacker.

Within the present context, the term "web regions" refers to topological regions of a piece of a web material, which are connected to each other and not separated or cut.

Web materials are often supplied in roll form, referred to when the width of the web defines essentially the width of the roll, or on spools, whereby the width of the spool is larger than the width of the web, and individual layers of the web are positioned adjacently albeit possibly overlapping in their y-dimension. Web materials may also be provided in boxes in a "festooned" arrangement, wherein one layer is folded onto the previous one, either in a single "accordion" arrangement or comprising a y-directional offset between individual layers.

Web materials need to satisfy certain requirements relating to their intended use, but they should further satisfy certain properties to allow or ease handling. Thus, webs should have a certain minimum integrity as well as bendability or flexibility, so as to allow handling. Webs may also need to satisfy certain properties to allow transportation thereof, such as certain minimum or maximum friction properties, porosities (i.e. resistance to fluids like gases when passing through), or electrostatic properties. Although any material does exhibit a certain inherent elasticity, webs are often referred to as "inelastic", when they are not intended to return to essentially their original dimensions after being significantly extended.

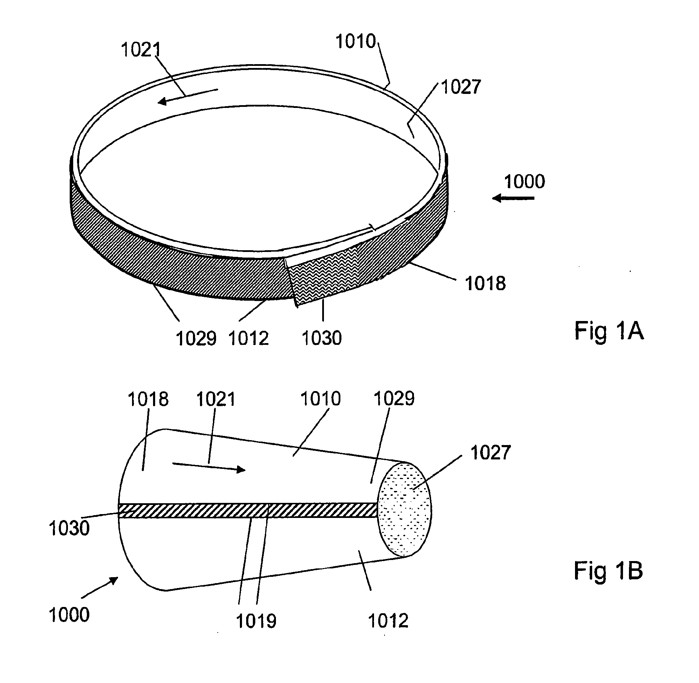

A shaped article according to the present invention comprises at least one piece of a web material and has either a three dimensional (3D) shape or is forming a belt like or hoop structure. Typical examples may be garments, such as a pair of pants, or girdles, or panty hoses, but also disposable hygiene articles, such as baby diapers, training pants, adult incontinence articles and the like. Articles may be combined to form a combination article, such as when a disposable absorbent structure is inserted into a reusable garment, such as menstrual pants. A first example for such an article is a belt-like structure 1000 formed of an elongated web, see Fig. 1A, here shown such that the front (1012) and rear (1018) end regions of a piece of web material 1010 (along the x- or machine direction 1021 of the web) are connected to each other in the connection region 1030. Similarly, as shown in Fig. 1B, tube-like articles 1000 can be made of an elongated web material 1010, when the opposed longitudinally extending lateral side margins 1019 are connected to each other in the connection region 1030.

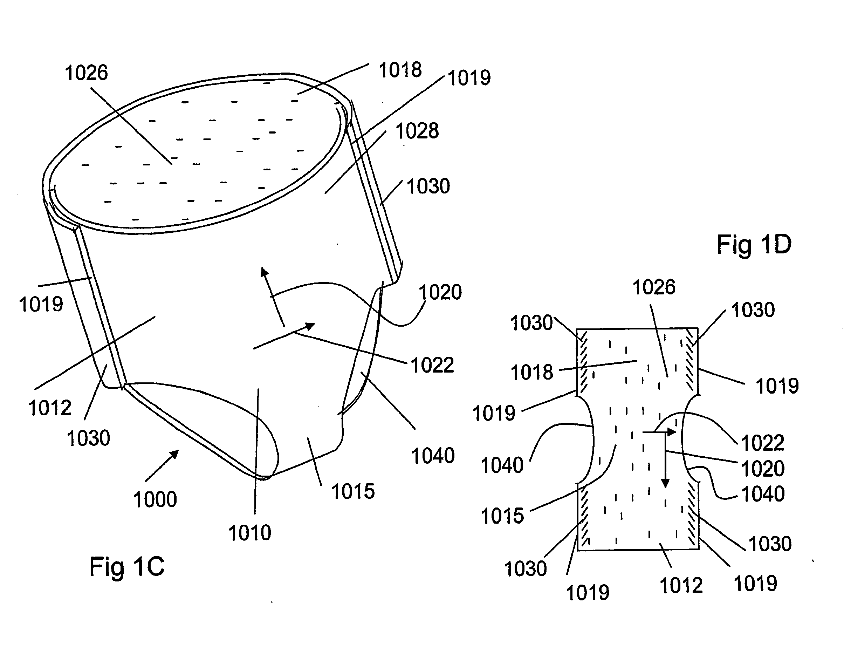

Also a simple pair of trousers 1000 may be formed out of a single piece of web material 1010, such as shown in Fig. 1C, where the front (1012) and rear (1018) regions are connected to each other at their side margins 1019 in the connection region 1030. In Fig. 1D, this article is shown laid flat after being cut open at the side margins 1019, thus showing a top view of the web piece 1010 onto the inner surface 1026, with a front (1012) and rear (1018) region and a connecting centre region (1015) with leg cut outs 1040. The straight side margins 1019 may or may not be parallel to each other. Also shown is the x- direction 1020 of the web material, and perpendicularly thereto the y- or cross-direction (CD) 1022.

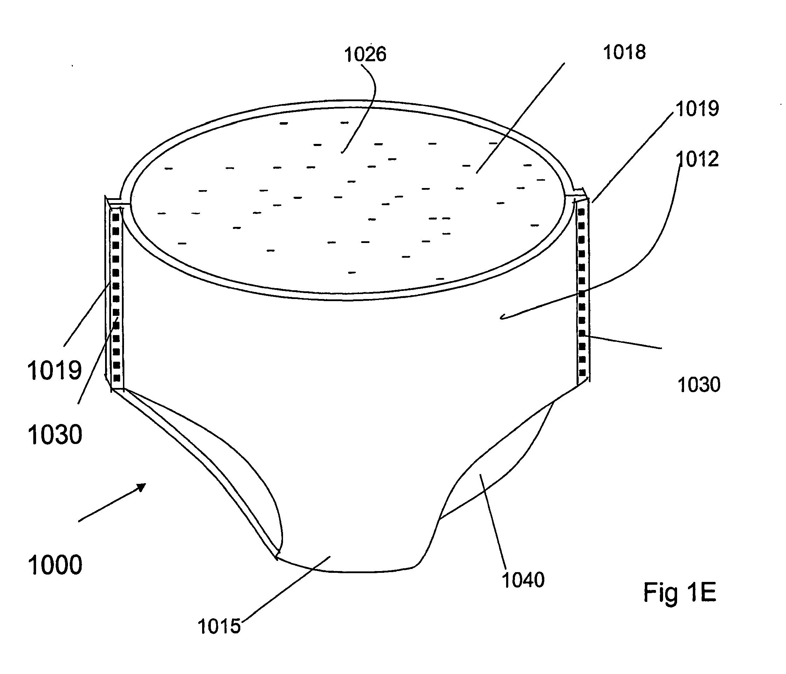

This design principle can also be applied to disposable absorbent articles in pant form as being worn around the lower torso of a wearer such as a baby or toddler, which are produced in a pre-closed state. As one alternative, these designs include an "overlap" arrangement of the front and rear parts, as schematically indicated in Fig. 1C, showing such an article 1000, having a front region 1012 and a rear region 1018 connected by the centre region 1015 which connects the first two regions through the crotch region of the wearer during use. In this "overlap" design, the connection is made in the connection regions 1030 between the front and the rear region, such that the first (inner) surface 1026 of one region (here the rear region 1018) overlaps the second (outer) surface 1028 of the other region (here the front region 1012). The manufacturing of overlap connections is well known in the art, such as disclosed in EP-A-0717972 or US-B-6481362. This is to be seen in contrast to a butt seam design, as schematically shown in Fig. 1E, which can be made by folding the article 1000 cross-directionally, such that the first (inner) surfaces 1026 of the front (1012) and rear (1018) regions contact each other and are then connected in the connection regions 1030.

For any such design alternative, the connection can be made permanent, such as by a line of adhesive, or by ultrasonic or pressure bonding, or it can be achieved by a re-closable "pre-closed" closure system. This refers to the fact that the article is shaped like a pant, and can be pulled up over the legs. When being worn, the closure system can be opened, such as for inspection by a care-giver, or for larger babies or toddlers when using a toilet.

The re-usable closure system may comprise re-fastenable adhesive tapes, mechanical fastener, or macro-fastener. Typically, the closure is achieved by positioning the front and rear ends of the unclosed article in an essentially flat state in close proximity to each other, and then to apply the connecting mechanisms. Often, an article comprises other elements, such as other (or secondary) web materials, strings, bands, or even other shaped elements.

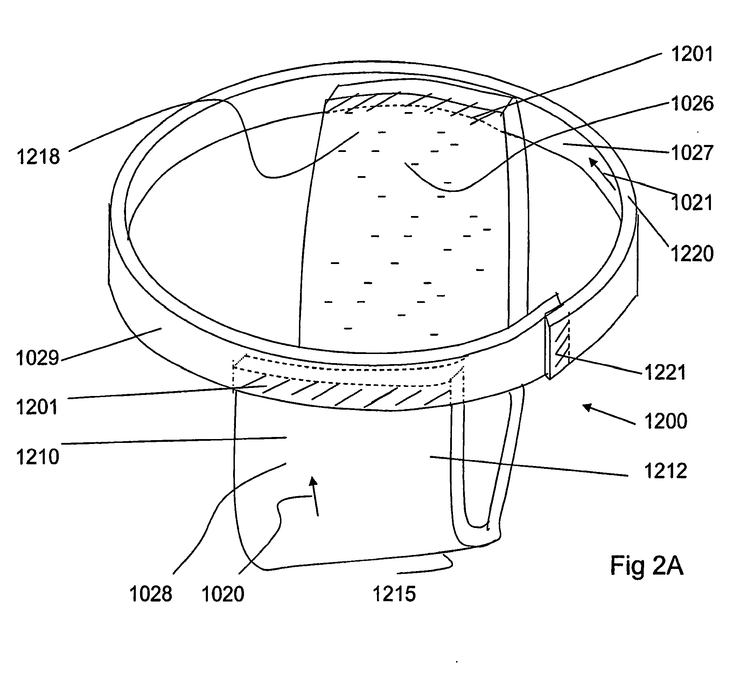

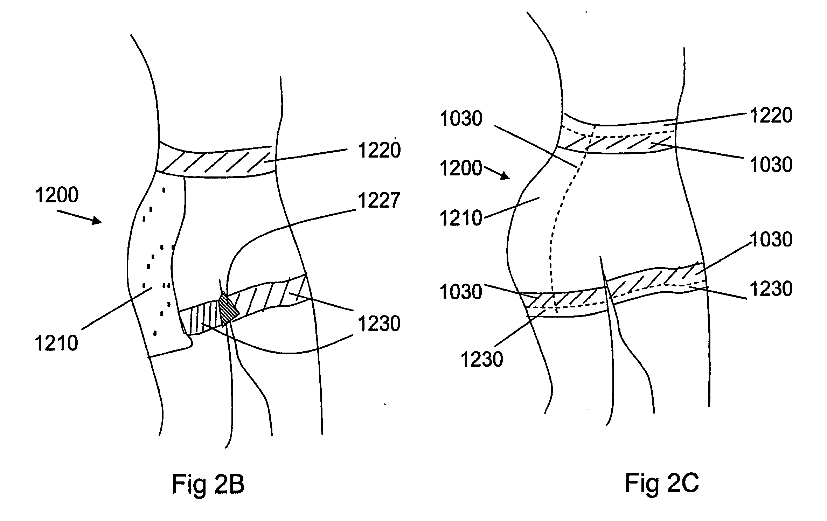

Shaped articles can also be formed of two or more web materials, or pieces or panels thereof, which are attached to each other, as schematically shown in Fig. 2 exemplifying a combination 1200 of a belt like structure 1220, such as shown in Fig. 1A, with a simple pant-like structure 1210. Therein, the two web materials may be arranged in a "CD/MD" fashion, i.e. if the machine (or x-) orientation 1020 of the article 1200 is defined along the "machine-direction" (or x-direction) of the web 1210, the MD-orientation (1021) of the belt like structure 1220 may be perpendicular thereto. This can be of particular benefit, if the belt-like structure is elasticised, which is easier to accomplish in the x-direction of this material. The belt like structure may be closed by a connecting region 1221.

The first piece 1210 of the article 1200 comprises a front region 1212, positioned in the front waist region of the wearer during use, a rear region 1218, positioned in the rear waist region of the wearer, and a centre region 1215 positioned in the crotch region of the wearer during use. In case of absorbent or hygiene articles, this piece of the article may comprise absorbent elements, and it may further be furnished with sealing components, such as leg elastics or leakage protection cuffs (not shown in the figures).

The two pieces may now be connected in the connecting regions 1201, such that the front and rear regions of the first piece 1210 are connected to the belt piece 1220 in the front and rear region thereof, respectively. In one embodiment as shown in the figures, a first (outer) surface 1028 of the first piece 1210 is connected in the front and in the rear to the second (inner) surface 1027 of the belt piece 1220, but it can be also that the inner surfaces 1026 are connected to the outer surfaces 1029 of the belt piece, or it may be different in the front and the rear. In yet an alternative design, the belt material may be a layered material, such as being folded along its x-directional axis, and then the first piece 1210 may be positioned between the two layers.

The front and rear regions may be connected in any conventional manner as known in the art, in particular with permanent or releasable connection systems. The first may be advantageous for non-disposable articles, or e.g. for disposable articles having a simple and inexpensive belt piece 1220. A releasable connection allows for designs with a re-usable belt piece, whilst the first piece may be disposable after each use.

Fig. 2B shows a protective garment 1200 having a centre piece 1210 connected to the rear portions of a waist belt 1220 and two leg hoops 1230. As described in US-B-4894869, the centre piece may be a liquid impervious and optionally thermally insulating material to protect the wearer when e.g. sitting on wet and / or cold places. The centre piece may have a cut in the crotch region so as to allow better fit in the inner thigh region, and may be provided with a gusset 1227. Instead of a gusset, the material may be deformably stretched in this region, e.g. by means described in US-B-5143679.

This design may be modified to form "three hoop pants" or trousers, such as when a second, front part of the centre piece is added. Either this may be a separate piece of web material, and the two pieces are at least connected in the crotch region, or it may be an extension of the rear part of the centre piece extending through the crotch region into the front region, as shown in Fig. 2C, with three belt structures, 1220 around the waist region, and 1230 around the legs, here positioned on the outer surface of the centre piece 1210. The front and rear parts of the centre piece may be connected to each other e.g. by a bond line 1030, and to the hoops by connecting regions 1230.

The present invention shows particular benefits when being applied to more complex articles, in particular when these have a "three-dimensional" (3D) shape.

An article has a 3D shape, if the article comprises at least two connected regions, or pieces, or panels and if the article cannot be laid flat on one plane with all seams or connecting lines also lying flat on this plane, essentially without deforming the materials.

In contrast, the structures as shown in Fig. 1 and 2 can be laid flat on a flat surface (e.g. a table) after the articles have been formed and folded.

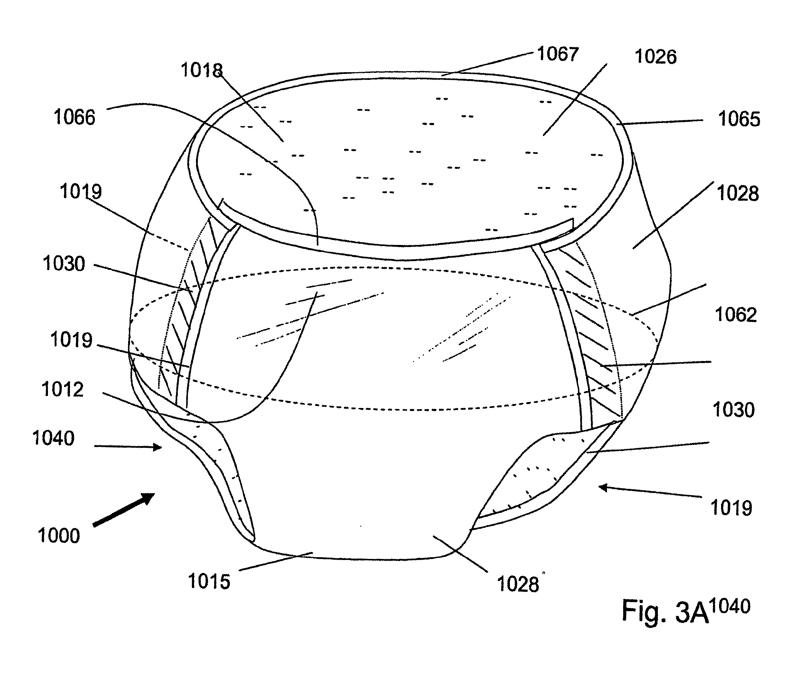

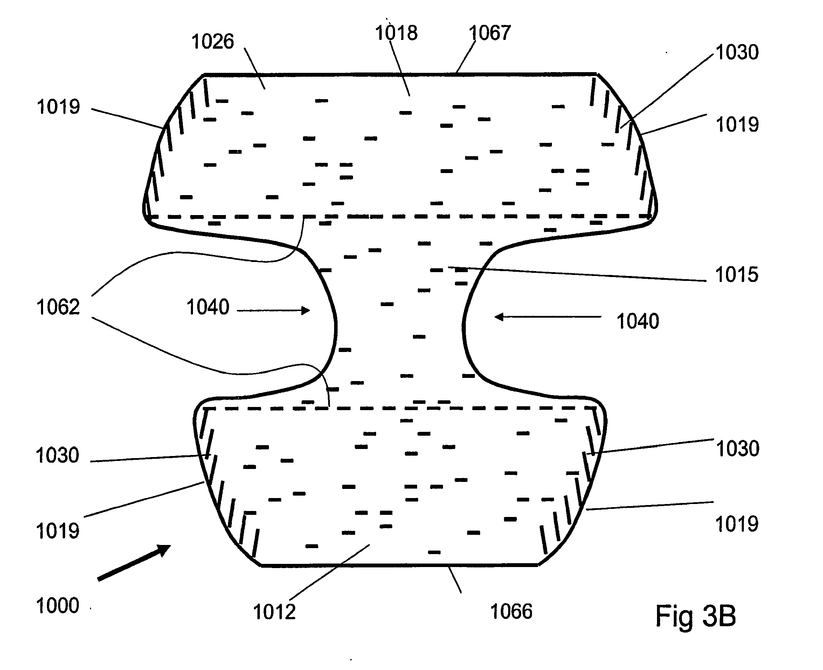

A simple execution of a 3D article is schematically shown in Fig. 3A and B.

This can be pants 1000, having a waist opening along a waist line 1065, and two leg openings along leg cut outs 1040. At both sides, there are connecting lines in the two connecting regions 1030, thereby defining a front region 1012 and a rear region 1018 and a crotch region 1015 connecting these two. Thus, the waistline 1065 has two sections, a front waistline 1066, here shown shorter than the other, rear waist line 1067. Fig. 3B shows a top view of the article cut open at the connecting lines 1019, which have a curvilinear shape and which delimit the connection region 1030. As shown here, the front and rear regions overlap, such that the inner surface 1026 of the rear region overlays the outer surface of the front region. Thus, the waist line 1065 is shorter than the hip line 1062, which is encircling the article (and hence the lower torso or the hips of a wearer during use) about at the upper perimeter of the leg cut outs 1040 (upward relative to the wearer).

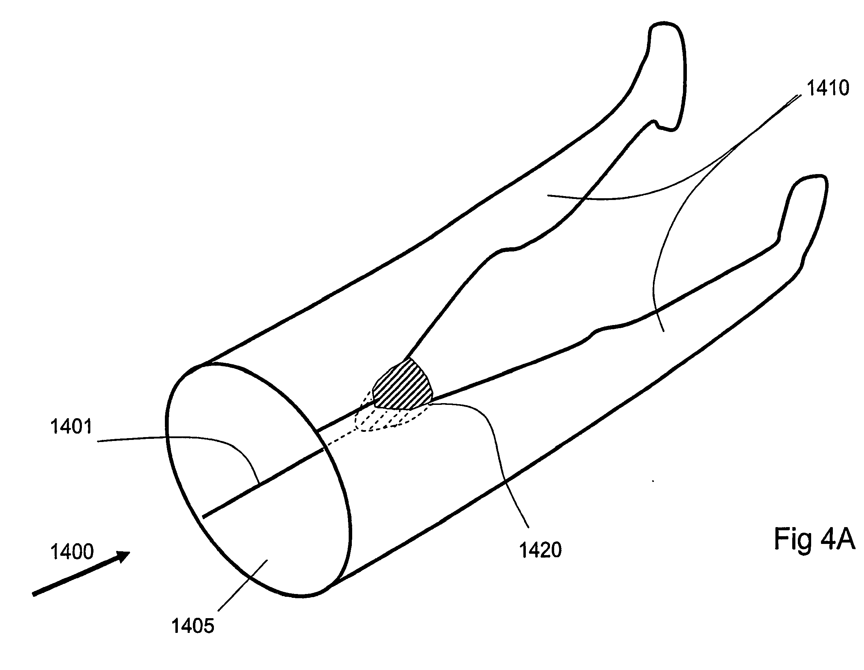

A further example of a simple 3D article is gored pantyhose 1400, as depicted in Fig. 4A, with two legs 1410, connected by bond lines 1401 at their upper (relative for a wearer) part to form a waist opening 1405. For improving the fit of the article, a gusset, or gore 1420 is inserted in the crotch region.

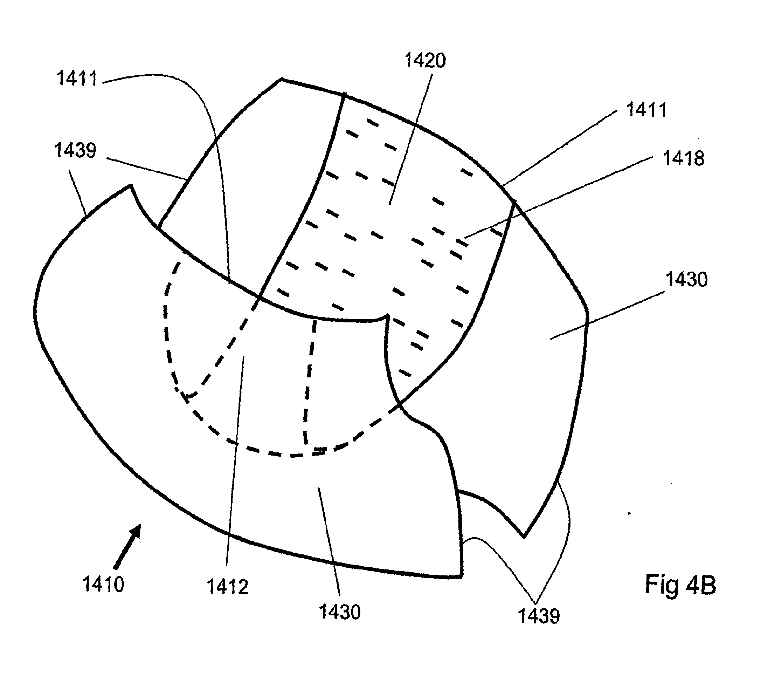

Yet a further example for a shaped 3D article is a skirt like structure 1410, such as depicted in Fig. 4B, showing schematically a disposable article. It comprises a first piece 1420, which may comprise absorbent and/or fit improvement or sealing means. The article shown in Fig 4B further comprises web pieces 1430 arranged outwardly the first piece, so as to form a skirt like structure, attached to the first piece in the front (1412) and rear (1418) part of the waist region 1411. These web pieces 1430 have curve-linear side margins 1439, which, when connected to each other in the connecting region, will create the 3D shape.

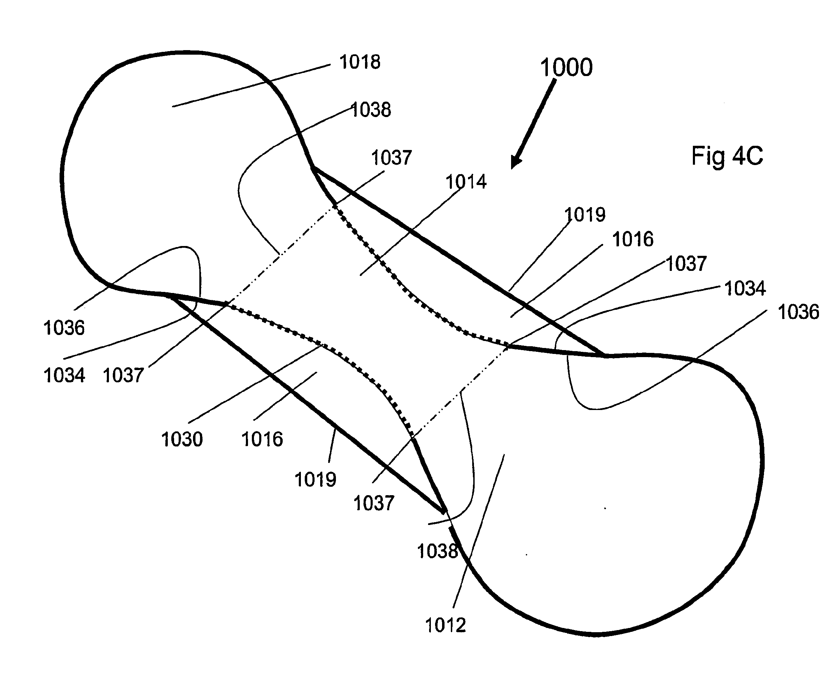

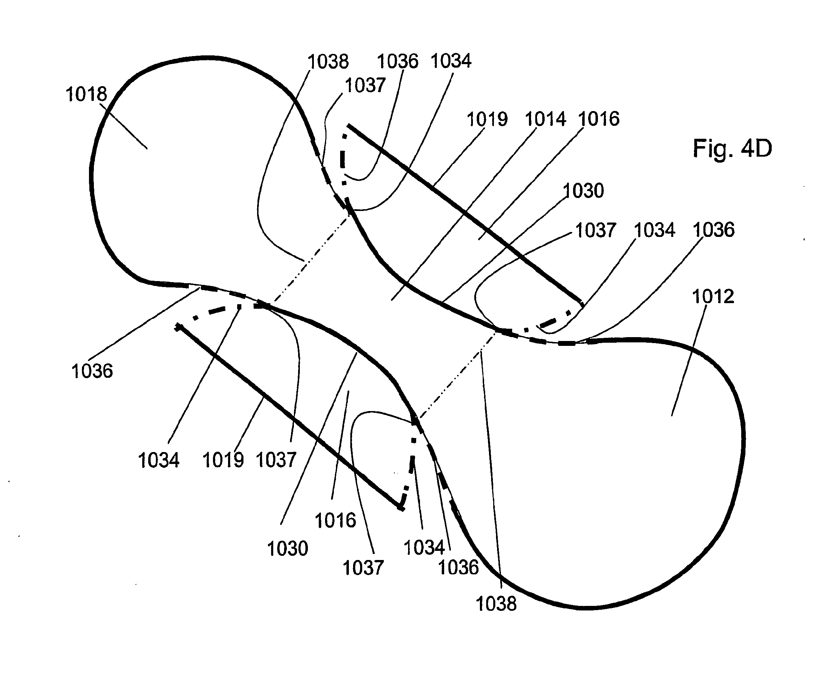

A further exemplary 3D article is described in Fig. 4C, showing a perspective view of a cup-like shaped pad, such as might be a feminine hygiene pad, and Fig. 4D showing a schematic exploded flat top view of the article 1000 with front (1012) and rear (1018) regions and a centre crotch region (1014) connecting these two. The side crotch regions 1016 may be unitary with the centre crotch region 1014, delimited by the curve-linear fold or connecting line 1030. As can be seen in Fig. 4D, this fold-line 1030 does not extend fully towards the laterally outwardly side margins 1019 of the side crotch region 1016, but terminates at an intercept point 1037. Looking outwardly from this point onwards, the side crotch region is delimited by a cut line 1034, and - when cut open and laid out flat - a semi-crescent cut out region between the side crotch region and the front and rear region appears. This cut-line 1034 is connected to a bond line 1036, extending from the intercept point 1037 essentially for - respectively rearwardly. This effectively foreshortens the laterally outward sections of the pad, and thus creates and maintains the cup-like shape. In this design, the centre crotch region 1015 is delimited from the front (1012) and rear (1018) regions by an essentially CD-oriented separation line 1038 connecting corresponding intercept points 1037.

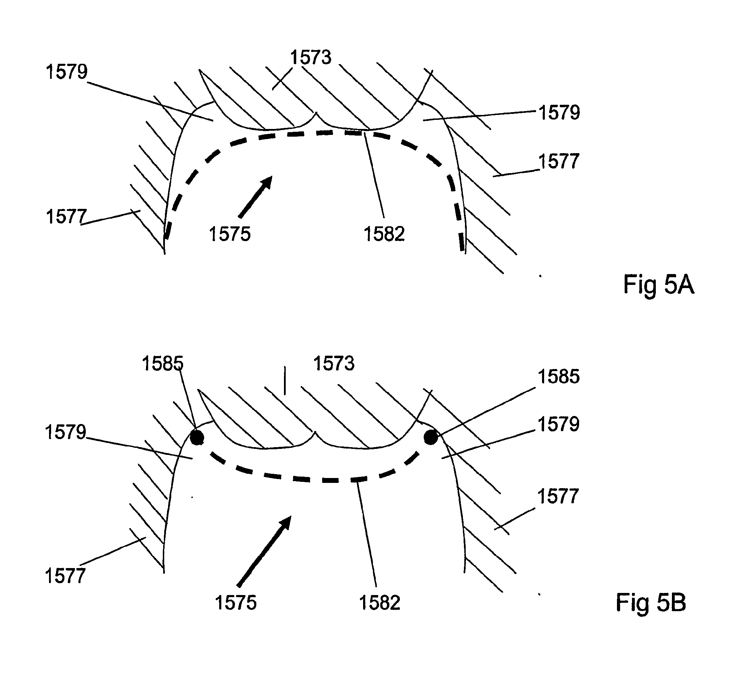

Another shaped article is a pair of trousers or pants with a leg seal cuff. Typically, articles like pantyhose fit the wearer during use tightly around the legs but, due to the elastic properties, less tightly in the crotch region of the wearer. In Fig. 5A, this crotch region 1575 is schematically depicted with legs 1577 and the lower torso 1573. The pantyhose will follow a schematic fit line 1582, close to the inner thigh region of the legs, close to the crotch region, but it would not fit into the crotch crease 1579 between the legs and the torso.

As shown in Fig. 5B, conventional diapers are typically designed such that sealing elements like elastics 1585 extend into the crotch crease 1579 without extending downwardly on the legs, such that the fit line 1582 follows a curved shape during use.

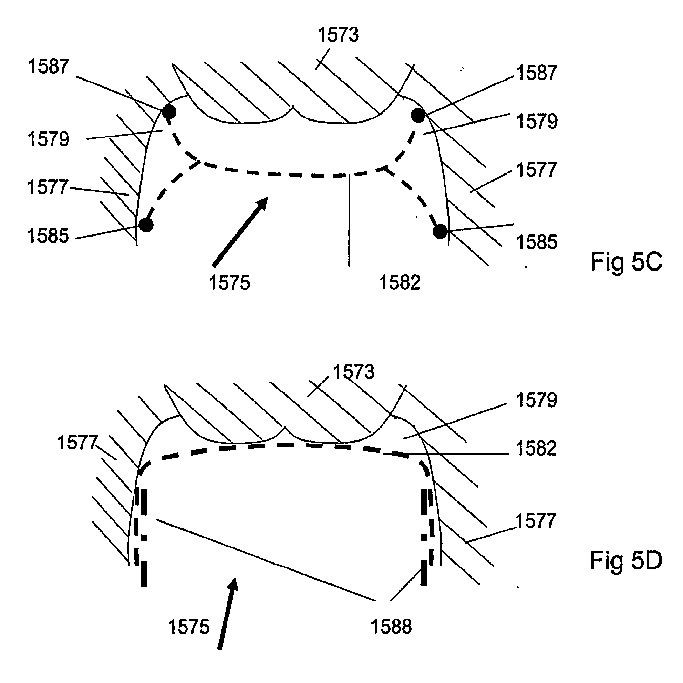

A further embodiment of a baby diaper comprises leg extensions in addition to barrier leg cuffs, also well known in the art as such, as described in more detail in US-B-6926702. For such an article (see Fig. 5C), the fit line 1582 connects the leg elastics 1585, now encircling the upper thigh of the wearer, with the barrier leg cuff elastics 1587, fitting into the crotch crease 1579. When considering the fit line of a pair of pants with three hoops (see Fig. 2C), it fits tightly to the thighs in the region of the elasticised hoops (see 1588 in Fig. 5D), but fits less tight in the crotch region.

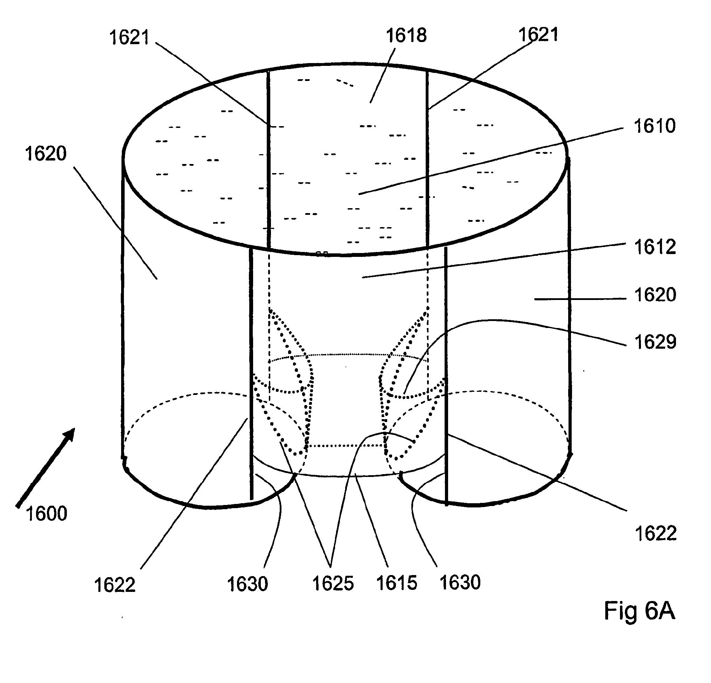

An alternative design is a pair of pants with leg seal cuffs, as depicted in Fig. 6A. A centre piece 1610 of the article 1600 extends from the rear waist region 1618 through the crotch region 1615 to the front region 1612. In the waist region of the article, side pieces 1620 are connected to the centre region by font (1622) and rear (1621) connection lines. In the crotch region extending towards the thigh region of a wearer during use, additional leg seal cuffs 1630 are connected along the downward continuation of these lines to the side pieces 1620. They are also connected to the centre piece, e.g. along connecting lines 1625, preferably executed in a curved shape.

The upper margin of the leg seal cuff 1629 is preferably executed in a curved shape, too.

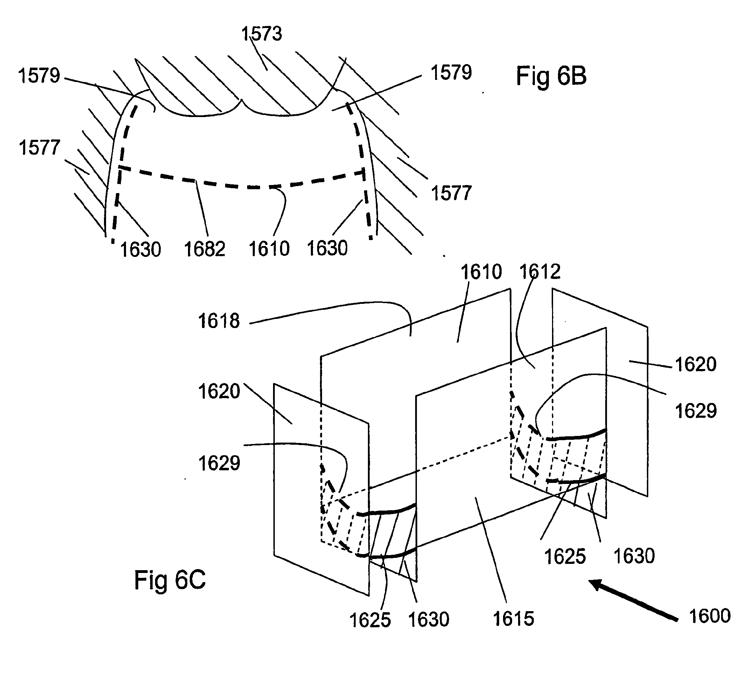

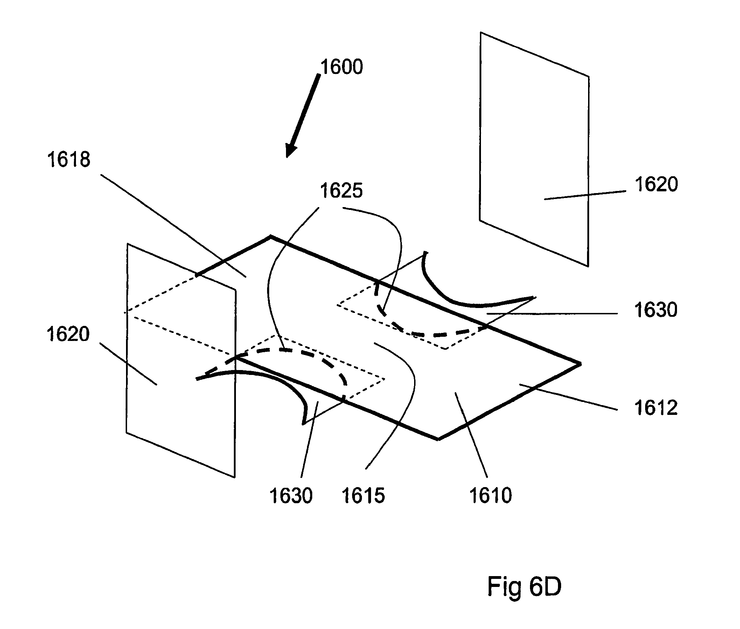

As can be seen in Fig. 6B, the leg seal pieces can lie flat on the thigh, so as to provide among other advantages significantly improved fit along the fit line 1682, here showing the leg seal pieces 1630 and the centre piece 1610 as elements thereof. Such articles are explained in more detail in co-pending application attorney docket ID EIP0207251, which is incorporated herein by reference in its entirety. Fig 6 C shows such an article in a partly exploded schematic view, and Fig 6D shows, how such a 3D article may be composed out of flat panels.

Such a leg seal cuff design provides particular benefits for articles, which require "acquisition space" (e.g. for diapers), tight but gentle sealing in the leg region (e.g. for feminine hygiene articles and especially menstrual pants), or gentle distribution of forces (e.g. for girdles), or provide a sustained dynamic fit for the wearer. There are various specific embodiments of this design principle, such as having certain regions of the article elasticized, e.g., the leg seal cuff may be elastically extensible in front-to-back direction only. Also, the side panels may be elasticized, optionally with a varying degree and / or directionality of elastication in different regions. The side panels may be openable, such as by combining a slit with a re-usable closure system, such as mechanical fastener. The side panels may also be composed of two sub-panels, e.g. first one made of a first material in the upper part (i.e. reaching into the waist region), and a second one made of a second material in the lower part (i.e. extending into the leg region).

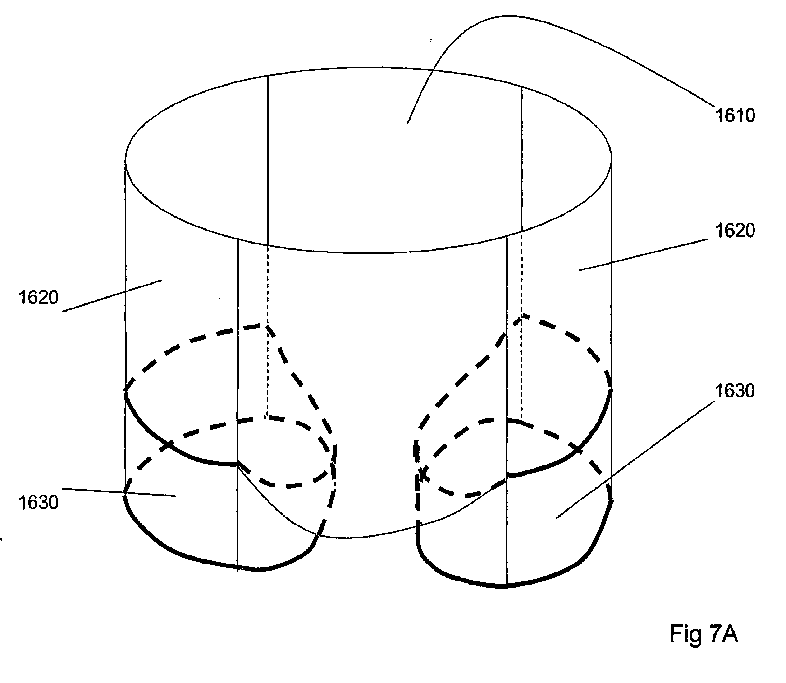

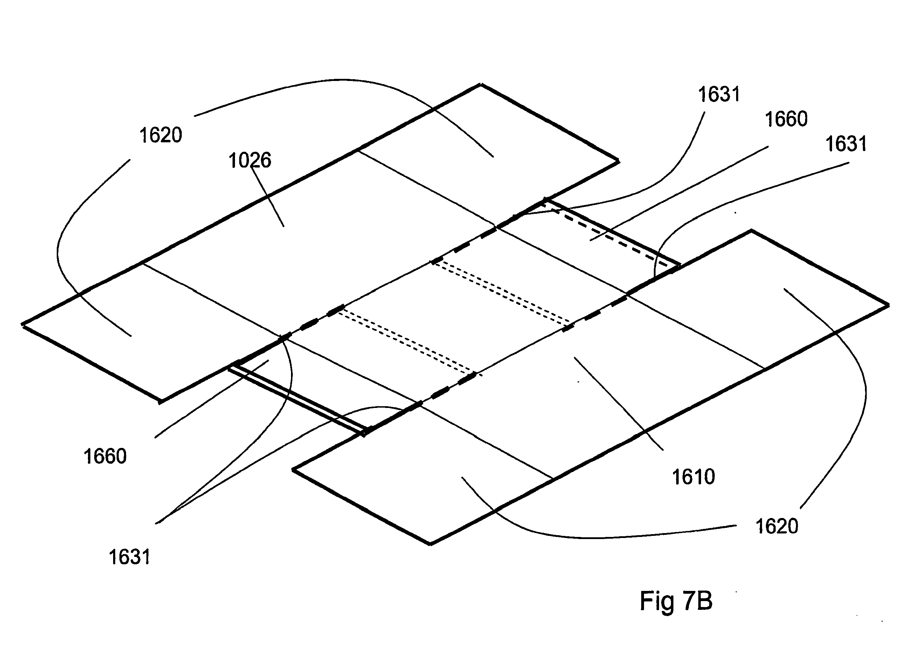

A particular variant relates to a design, for which the leg seal cuff extends from the inner thigh region all around the leg, thereby forming a hoop as described for the three hoop pants (see Fig. 7A and B). The leg hoops are here produced out of two layers of material, which are connected by bond lines 1631. Thus, such a design would also allow the outer hip portion of the pant to be composed out of two different materials - one extending to the waist region and the waist opening, and the other one extending to the leg opening.

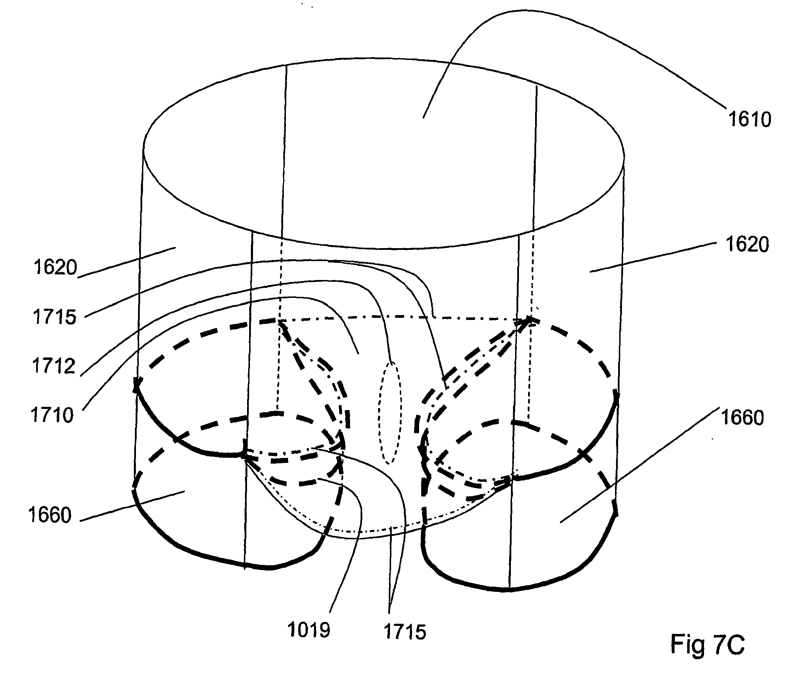

A particular execution of this variant is further depicted in Fig. 7C, which may be a disposable absorbent article, such as a diaper, being particularly suitable in the case of being loaded with faecal material. The design is analogous to the one as explained in the context of Fig. 7A and B. In addition, a so called secondary topsheet 1710 is designed to closely fit on the skin of the wearer, having an opening 1712 in registry with the anal opening of the wearer during use, and being adapted to allow the faecal material to pass through into a void space being maintained between this secondary topsheet and the liquid absorbent core. The secondary topsheet may be attached to the front and back regions of the centre piece 1610 along connecting lines 1715. It may also be connected to the inner sides of the inner leg cuff pieces, such that opening 1712 is kept under slight tension, hence open when the product is in use.

Any of the materials may be "breathable", i.e. allowing air but not liquids to penetrate through. The centre piece may include absorbent material or it may be combined with an absorbent core. The leg seal cuffs may have a certain liquid or moisture absorbent capacity, which may be designed to capture liquid leaking from the centre piece absorbent structure, or which may be designed to absorb sweat.

Whilst the above examples illustrate important application areas for the present invention, there exists a plethora of other articles, which may be conveniently produced by applying the present invention, such as a shirt, blouse, jacket, coat, pyjama, corset, brassiere, girdle, or other protective and optionally disposable garments like surgical gowns, overall, masks, hats, gloves, sleeping bags, garlands, etc., including semi-finished items for any such articles.

As can be seen in the examples of shaped articles, such articles can be described by an "inner"

(1026 / 1027) and "outer" (1028 / 1029) surface, as depicted in Fig. 1 and 2, wherein each of these surfaces corresponds to the first or second surface of the web material, from which the article is formed. If, for example the article is worn, then the inner side is oriented towards the wearer, and the outer surface is opposed. However, such an article may be "inverted", e.g. seamed garments may be produced "inside out" and then be turned or inverted. Thus, the first surface of a web material may be the inner surface of the article during manufacturing, but may become the outer one during use, e.g. after such an inverting. Particular designs are "symmetric articles", for which "inner" and "outer" side look essentially identical.

Similarly, such articles can be described by a space, which is "inside" of the article, and the complementary space, which is outside of the article. The "inside space" is thus enclosed by the inner surfaces and - if these do not form a closed space - by one or more hypothetical cover(s) closing the space. Thus for a cylindrical tube, the cover will be represented by the circular areas at the front and rear edges. More generally, for 3D shaped articles of more complex shape, inside refers to the smallest space enclosed by the inner surface of the article and the smallest cover area required to close the space. Hence, for a pair of trousers, the inside space is formed by the inner surface of the article and the areas of the waist opening and the two leg openings. For any cup- or bowl-like saped (or concave) article, the inner space will correspond to the volume of the bowl, whereby the surfaces will be complemented (if required) by the smallest closing areas. It should be noted, that the term "inside space" applies to the formed article, however, if one or more web materials are positioned so as to form an article, but are not necessarily connected yet (i.e. still forming unconnected pieces), this "preformed" arrangement is considered to have a corresponding inside space as just described.

The shaped articles as described above comprise web materials or regions thereof that are connected to other materials or regions. In the present context, "connected" refers to any physical or chemical connection between two pieces of material, or regions of one piece of material. Thus, it includes permanent attachment or bonding, such as welding, gluing, sewing, etc. Optionally such permanent attachments may have features to ease destructive tearing of the attachment, such as may be desirable for disposable pant-like absorbent articles. The connection can also be non-permanent, such as by using re-usable adhesive tapes, so-called mechanical fastening devices comprising connecting loop and hook structures, or so called macro-fastener, such a slot and tab designs, such as described in US-B-6669618. Typically, this re-usable attachment is closed during manufacturing, and the user may open it during use, such as when a caretaker applies or removes pant-like absorbent articles to or from babies or toddlers.

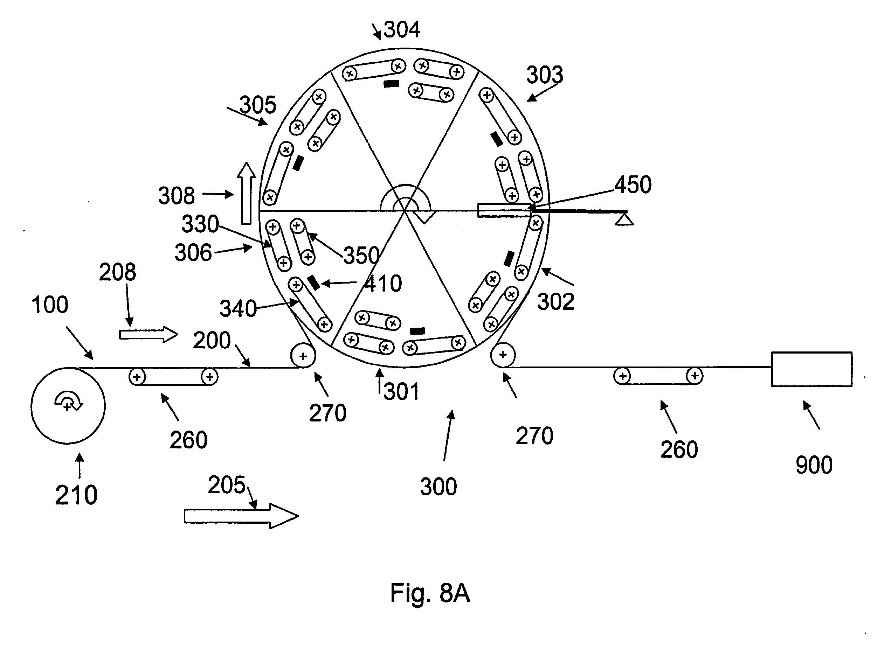

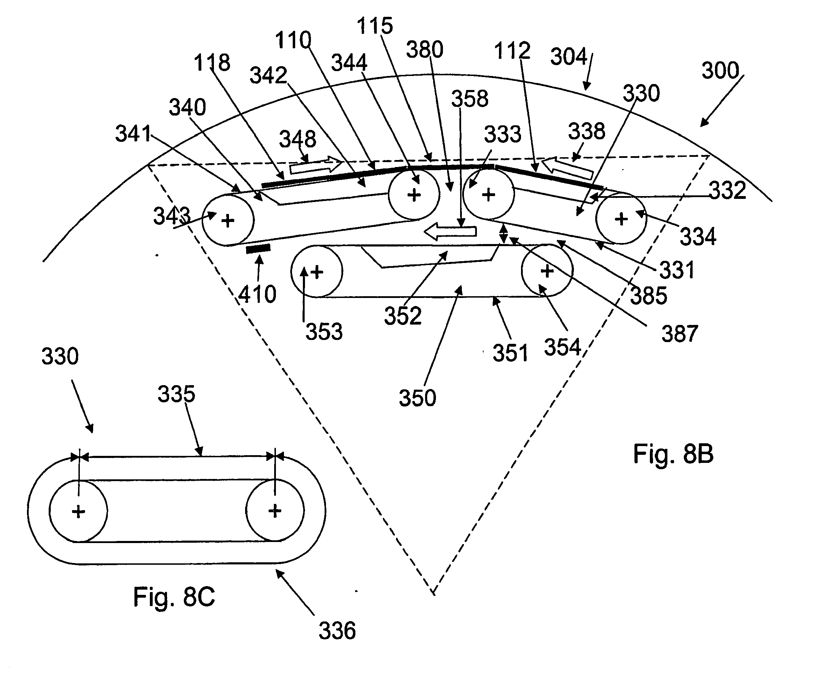

The present invention provides a simple and effective solution to producing shaped articles, such as belted, or "hooped", or 3D articles, such as described hereinabove. The present invention applies the principles of web handling as laid out in detail in co-pending application PCT/IB05/000845. As shown in Fig. 8, it is a method and equipment for handling a web material 100, which may be an essentially continuous web or an essentially continuous sequence of pieces of an essentially continuous web. The web material runs on a web handling equipment having a web path 200 connecting a web supply means 210 with a process section end point 900 along the overall web path direction 205. The web material comprises at least a first and a second region, whereby the first region is oriented along the web path more towards the process section end point 900 than the second region.

The method comprises the steps of
a) providing a web material 100 on a web supply means 210;
b) moving the web material 100 from the web supply means 210 towards the process end section 900 along the overall web path 200 at an overall web path speed |v₀| (208) relative to the frame of the web handling equipment;
c) providing a web path splitting means 300 positioned along the overall web path 200 and comprising at least a first and a second web handling section (301, ..., 306), each of these web handling sections comprising a section frame and a web support means (330, 340,...) connected to the section frame, having a surface which is movable relative to the section frame;
d) splitting the web path on the web path splitting means 300 into at least a first and a second web sub-path, each running through one of the web handling sections; and transferring the web material along the web sub-paths to the web handling sections;
e) handling the web material of the web handling sections by
   e1) affixing the first region 112 of the web material to the surface of a first web support means 330 in the initial contact region 335 of the web support means without affixing a second region 118 of the web material thereto;
   e2) changing the speed of the surfaces of the web support means 330, while having the first region of the web material remaining affixed thereto, thereby changing the relative speed of the first section 112 of the web material to a second section 118 of the web material;
f) thereby transferring at least parts of the web material out of the initial contact region 335 of the first web support means 330 into an operating region 336 of a web support means 330 or of a further web support means, and thereby forming a cross-directional fold in the web material;
g) performing one ore more web treatment steps on the web material by means of a web treatment unit, whereby at least one treatment step is the connecting of web materials;
h) removing the web material from the web handling section;
i) providing the web handling section for repeated executions of the web handling steps d) to h).

An apparatus to execute such a process comprises
i) a means for supplying (210) and for transferring (260, 270) the web material 100 towards a process section end point 900, thereby defining a web path 200 for transporting the web material at an overall web path speed 208;
ii) a web path splitting means 300 positioned between the web supply means 210 and the process section end point 900 for splitting the web path 200 into at least a first and a second web sub-path;
iii) the web path splitting means 300 comprising a web handling section (301, 302,..) for each of the web sub-paths for handling the web material 100;
iv) each of the web handling sections (301, 302,....) comprising a section frame and at least one web support means (330, 340, ...) connected to the frame,
wherein the web support means has or have
a) a web support means surface for temporarily affixing a region of the web material to the web support means (330,...),
b) an initial contact region (335, ...) and an operating region (336, ...);
c) a web support drive means (333,...) for changing the speed of the web support means surface of the web support means 330 relative to the frame of the web handling section, thereby moving at least a portion of the web material from the initial contact region 335 into the operating region 336, for creating a cross-directional fold of the web material;
v) and a web treatment unit comprising at least two web treatment elements, at least one treatment head 410 and a treatment tool 450 there shown to be affixed to a base outside of the rotating drum 300.

The web support means 330, ... may be connected stationary relative to the treatment section frame, or may be movable, such as translatorily, such as indicated with arrow 357 for web support means 350, or rotationally, such a pivoting.

The web path splitting means may be a rotatably mounted drum 300. As shown for one exemplary web support means 330, this can have an essentially endless surface, preferably a belt 331, an electrical drive means 333, preferably an electrical servo motor, and a means for temporarily affixing the web material to the web support means, e.g. electrostatics, or mechanical fixation means, or preferably vacuum suction means 332. In a preferred embodiment, the web support means is a belt system, with an essentially endless belt, having a freely programmable electrical drive means integrated in a belt support roll, such as Smart Motors^{™} SM1720 R, such as available from Animatics, Santa Clara, CA, USA, preferably employing non-contacting data and power supply such the CombiTrans system of Gauss, Salem, Germany. The apparatus may further be equipped with a helical screw feeder system adapted for receiving web material pieces for positioning of such pieces in a stack.

The web treatment unit of the present invention comprises at least two treatment elements, such as a treatment head 410 and a treatment tool 450. During the treatment step, the two elements are positioned on opposite sides of one or more layers of a web material. The web(s) are positioned on the treatment head 410 by at least a first and a second web support means 330, 350, which are arranged such that the distance 387 of the gap 385 between them can be varied, and the treatment head can be positioned in this gap 385 to treat the web.

Thus, the present invention allows the particular treatment of a web material. The term "treatment" refers to any physical or chemical interaction of a treatment unit with the web material, whereby the web material(s), or pieces or regions thereof are affixed to an element of the treatment unit.

For example, in order to connect two material pieces, these pieces must be appropriately positioned relative to each other, before they can be connected. Thus, the treatment unit has the function of positioning and connecting, such as by applying adhesive, or by thermal welding.

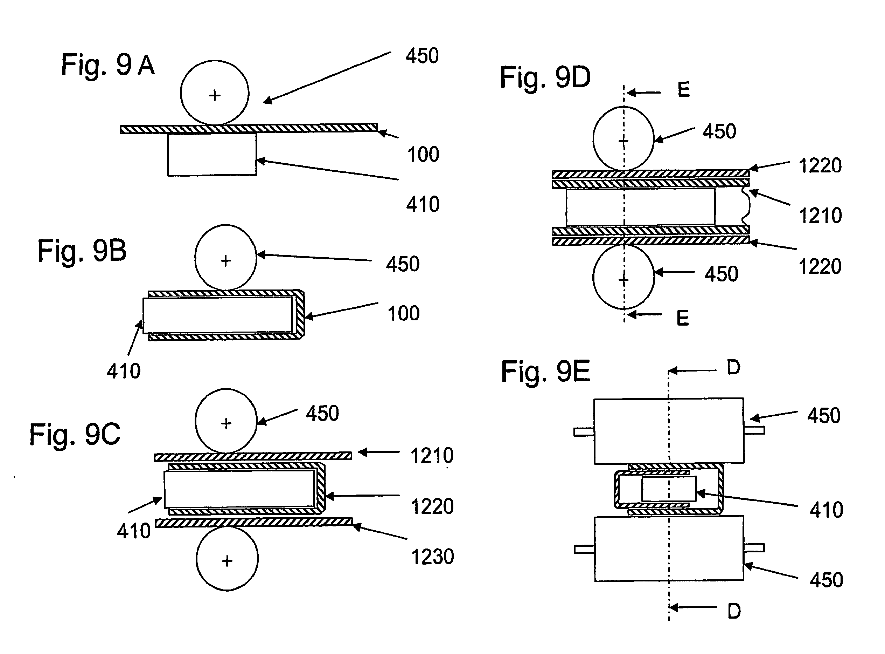

Fig. 9 exemplary and schematically depicts various options for arranging treatment elements with regard to the positioning of the web. Fig. 9A and 9B show the treating of a single layer of a web 100 by a treatment head 410 and a treatment tool 450. Fig. 9C shows the treating of three webs 1210, 1220, and 1230, e.g. by connecting these by means of a treatment head 410 and a treatment tool 450. Fig. 9D and E show the connecting of two webs 1220 and 1220 to form a closed structure. Thus, there may be one or several regions of one web being attached to a treatment head, but there can also be several regions of several webs being attached to the treatment head. Thereby, the different regions may be in a fully overlaying arrangement, or partly overlaying each other. For example, two web regions of two web pieces may be positioned non-overlapping next to each other and both may be combined with a third web piece.

Within the context of the present invention, a "treatment head" is a treatment element, which can be positioned inside the article (respectively inside the web material which will form the article), whilst a "treatment tool" is the counter element to the treatment head.

For example, the treatment head may ensure appropriate positioning and supporting of the material whilst a treatment tool such as a heated embossing roll, operates on the "outside" (opposite surface) of the article. The treatment tool outside of the article may also be a counter pressure plate, whilst the treatment head moves the material against this plate.

The treatment unit may be a unitary piece of equipment having the two treatment elements connected via arms, one for each side of the web. The treatment unit can also comprise several essentially separated treatment elements cooperating appropriately for the treatment. The treatment unit can also comprise one treatment head on one side interacting with more than one treatment tools on the other side.

For the example of the web path splitting means being a rotating drum (or turret) with several treatment stations, each of these stations will have at least one treatment head. Each of these treatment heads may cooperate with a treatment tool affixed to the frame of the same treatment station. There may also be just one treatment tool affixed to the frame of the turret (i.e. it does not rotate when the turret does). As schematically depicted in Fig. 8A, the treatment heads 410 rotating with the turret may pass by the (relatively stationary) treatment tool 450. Similarly, there may be multiple treatment heads per treatment section, interacting with one treatment tool per head, per section, or per web path splitting means. Further, there may be several treatment tools with a different functionality cooperating with one treatment head. Thus, there might be in each treatment section one treatment tool for connecting one set of regions, and a second treatment tool connecting a different set of regions. Similarly, there might be several stationary tools cooperating at different positions with one treatment head when this passes by due to the rotation of the turret.

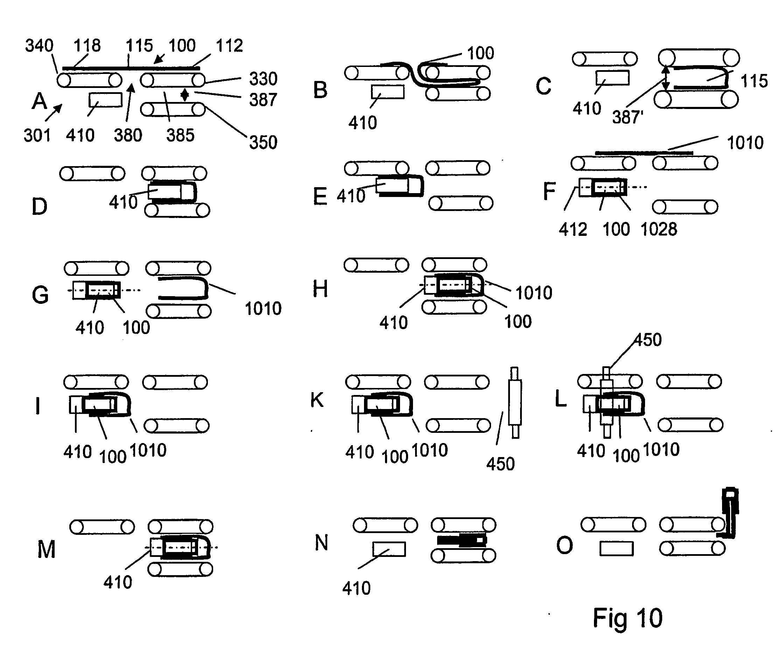

The interaction of the various elements is schematically shown in Fig. 10, showing an exemplary sequence of process steps A to O for connecting the front 112 and rear 118 regions of a first piece of material 100 by a second web material 1010 in a web treatment section 301. First (see Fig. 10A), the first web material 100 is transported to a first web support means 330, here overlaying a further, optional web support means 340. The surfaces of the web support means with the web material regions attached thereto, are moved against each other (Fig. 10B), causing the centre region 115 to fold and to move through gap 380 into the gap 385 between the first (330) and a second (350) web support means. The gap distance 387 between the first (330) and third (350) web support means is now increased (Fig. 10C), here shown by moving the second web support means 350 downwards, whilst the front and rear regions remain firmly attached to the web support means surfaces, and the centre regions 115 compensates for the increased distance. The treatment head 410 is now moved into the opened gap between the front and rear regions (Fig. 10D), and the web material is transferred to the treatment head 410 (Fig. 10E). Further, the treatment head is moved out of the gap 385, and rotated 90° around its longitudinal axis 412, such that in Fig 10F the outer surface 1028 of the web can be seen. Now, the second web material 1010 is opened analogously to the first one, and Fig. 10G shows this piece 1010 attached with its front and rear regions to the web support means 330 and 350. Then, the treatment head 410 with the first web 100 attached to it, moves again into the gap 385, and can now receive the second web 1010 on the free surfaces not covered by the first web 100 (Fig. 10H). The treatment head can then move out of the gap again (Fig. 10I), such that the sides or bevelled corners allow the webs 100 and 1010 to overlay thereon (not shown as such) whilst having sufficient room to interact with a treatment tool 450. As shown in Fig. 10K, the treatment head 410 is approaching the treatment tool by the continuing rotation of the rotating drum towards the stationary treatment tool 450, such that the webs may be connected by heat or pressure bonding (Fig. 10L). This can be completed by the interaction with several treatment heads for different connecting or treatment regions (not shown). When the treatment is finished, the treatment head is moved into the gap 385 again (Fig. 10M), the connected webs are transferred to the web support means again, which may close the gap distance 387 (Fig. 10N), and the connected webs may be transported out of the equipment, e.g. to a packaging station (Fig. 10O). Care should be taken with the last step with regard to avoiding overly compressing the materials, if these now have a 3D shape, which would allow folding only with deformation of the webs or connecting lines.

The two main functions of the treatment unit are the accurate positioning of the web material(s) for the treatment, and the treatment itself. The positioning may be achieved by the treatment head alone or in cooperation with the treatment tool, whilst the treatment itself will always require interaction of the two elements.

The positioning may be achieved by means, which releasably affix the web thereto, such as by increased friction, or releasable adhesives or the like, or as a preferred embodiment, vacuum suction. In a preferred embodiment, the treatment head, i.e. the treatment element to be positioned in the web opening, will position the web accurately.

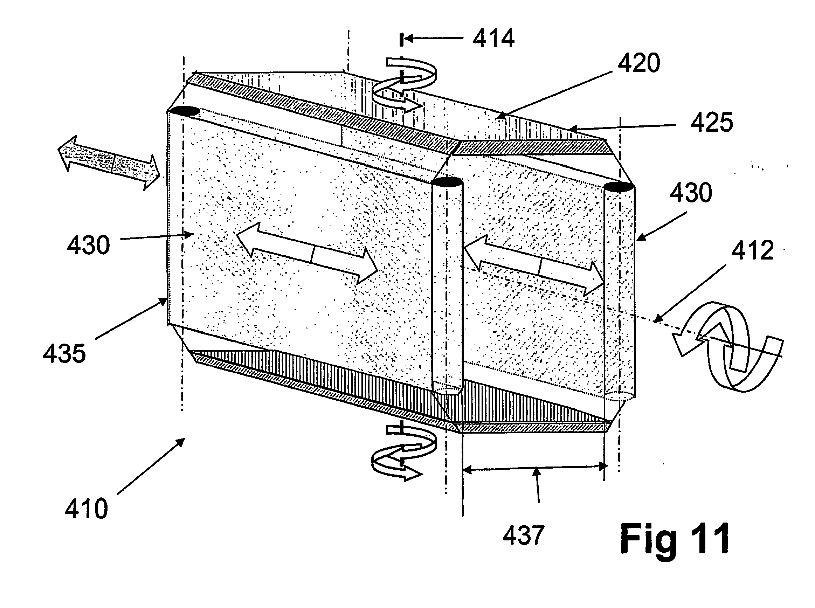

The positioning of the web by the treatment elements may be achieved by various movements, which the treatment element may undergo. Fig 11 schematically shows in an exploded view a treatment head having two web receiving surfaces 430 in an opposing arrangement, and two other surfaces 420 of cover plates 425. The treatment head may be affixed to the frame of the web treatment section (not shown), and the movement is described relative to this frame. The head may be transversely movable along a longitudinal axis 412, corresponding to the longitudinal axis 412 in Fig. 10D and F, so as to move the treatment head in and out of the gap. The head may also be mounted to rotate around this axis. The treatment head may also be mounted rotatably around any other axis, here shown axis 414 perpendicular to the longitudinal axis 412. Axis 414 may also be positioned such that it does not run through the treatment head, such that upon rotation around this axis the treatment head may be swivelled, e.g. by 90°. Similarly, it may translatorily move along any of these axes, and it may move curve-linearly along any suitable trajectory path.

In a particular embodiment, the treatment head is equipped with moveable surfaces, such as shown in Fig. 11 by showing transport belts 435, the design of which may follow the principles as laid out for the web support means as described hereinabove. The four surfaces of the treatment head may be in a relative stationary positioning, or they may move relative to each other. For example, the distance 437 between two opposing surfaces 430 may change during the process.

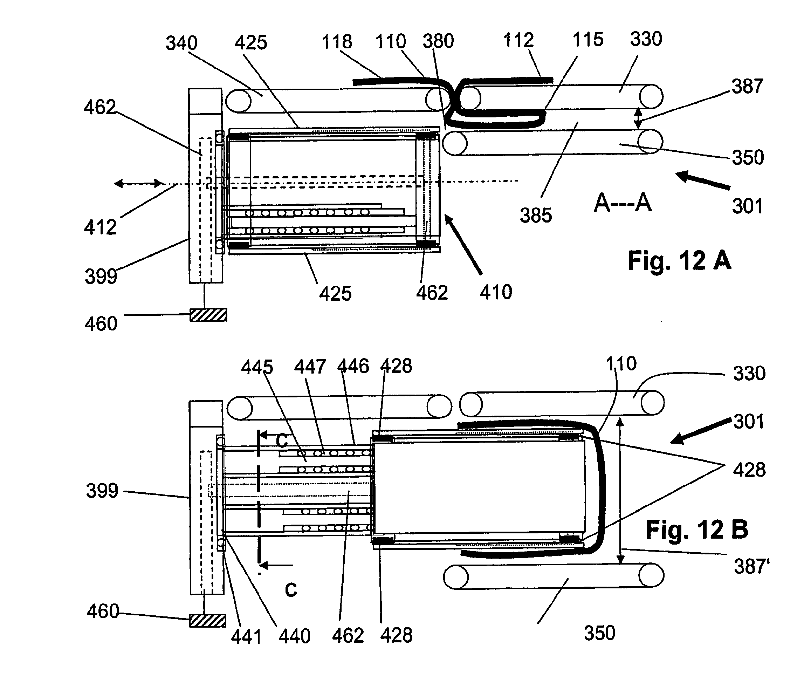

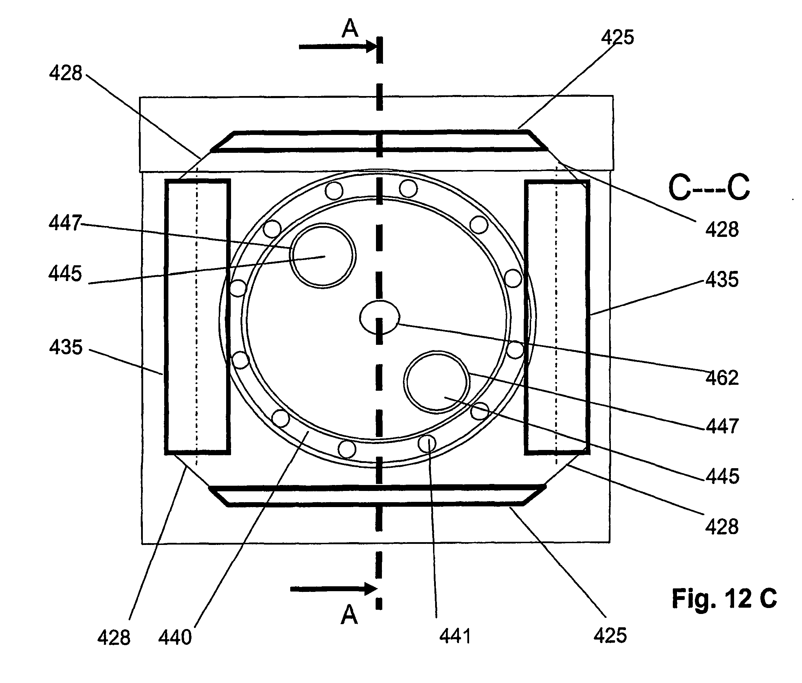

Fig. 12 shows a schematic view of a treatment head 410 in a web treatment section 301 of a rotatably mounted drum or turret, comprising a web treatment section frame 399, a first web support means 330 and a further web support means 340 positioned radially outwardly, and a second web support means 350, which is moveable radially (or z-directionally relative to a web 110) to adjust the width 387 of the gap 385 - compare Fig. 12A showing the web 110 with its front (112) and rear (118) regions as well as centre region 115 being moved into the gap 385 at a gap distance 387 and 12B showing the gap distance increased (387').

The treatment head is similar to the one shown in Fig. 11, having two opposed cover plates 425 and two opposed belt systems 435 rectangularly arranged.

The belt systems may be driven by freely programmable drives, as described for the web support means hereinabove, or by other appropriate drive means. The belt systems may also have a vacuum suction system, as may be supplied by a vacuum source 460, delivered to the rotating drum by conventional means to a the web treatment section frame 399, and via a flexible tube system 462 to the lateral end of the treatment head, from where it may be further distributed to the transport belts 435. Optionally, the cover plates 425 may also be connected to the vacuum source, such that a web may also be affixed thereto. The cover plates and the transport belts may be connected by connection system 428, optionally designed so as to allow adjustment of the distance between the belt systems. The treatment head is affixed to the frame by a rotatable base ring 440, here shown with roller bearings 441. Further, the treatment head comprises a reciprocating piston system 445, here indicated by three tube- or rod-like elements 447 and roller bearings 446. The piston system may be driven by freely programmable linear motor drives 448, a cam based drive system, or a vacuum and positive pressure based system, or other appropriate means. Whilst the treatment head is shown affixed to one side of the frame, it may also be affixed on the opposite side thereof, or in any other manner allowing to move into the gap between the first and the second web support means.

Apart from the positioning and optionally re-positioning of one or more webs pieces or regions, the treatment unit will have special means to interact with the web.

A simple interaction may be the folding of a region of a web along a longitudinally oriented folding line by means of a folding plough, such as when lateral side regions of a web either extend beyond the side margins of a web support means, or are released from a web support means, such that these can be folded along a longitudinal fold line. Such a folding plough can be mounted stationary on the equipment frame or on the section frame. Typical other interactions may be connecting by gluing, welding, compressing. etc.. It may also include the attaching of connections means like buttons or macro fastener. Another typical interaction may be the cutting of webs, such as by rotating knives, laser beams, etc.. A particular interaction relates to the ability of the treatment head to change its size, respectively its circumference, as described hereinabove. This can for example be used to receive an elasticized web in an non-expanded state, but to further treat it in a stretched state, e.g. bond it in stretched state against another web such that the combined web will contract when released. In case the circumference of the head is controlled by respective control means acting in cooperation with appropriate sensing devices, this circumference variability can be used to precisely position different materials relative to each other. Also, several treatment steps may be executed in parallel or consecutively by one treatment unit.

During the treatment (other than potentially the positioning or repositioning), the treatment elements of the treatment unit will cooperate, with one on each side of the web. Cooperating can mean, that one element functions as (counter-) support whilst the other acts on the web, such as by pressing, or spraying, embossing, etc.. Both elements may have intermeshing tools, e.g. rolls, such that the web between these rolls is stretched (see above mentioned US-B-5143679). One or both of the elements may provide temperature changes (heating / cooling), changes in position in x- and y-direction thereby creating a specific glue pattern while passing by a stationary glue head.

The web support means need to position the web materials or articles accurately relative to the treatment head, and the article must be "opened" so as to allow the treatment head to be positioned "inside" the article. To this end, at least one of the first or second web support means is moveable relative to the other so as to "open" the inner space of the article (or the web materials or pieces thereof which are about to form the article during the process).

In the case of the web path splitting means being a rotating drum with web treatment sections as segments thereof and web support means being connected to the mounting frame of the web treatment section segments, the second web support means may move "up and down", which typically will be relative to the x-direction of the web material, or inwardly / outwardly relative to the frame of the rotating drum. The inner space may also be opened by pivoting the web support means appropriately (typically around an axis parallel to the y-direction of the web, and hence of the web support means).

Once the inner space of the article is accessible to the treatment head, the head will be re-positioned into this space. This can be achieved by any relative movement of one or more web support means and the treatment head along any three-dimensional path, such as translatory movement along a straight, curve-linear, or a circular path. It may include rotational movement, or pivoting around any axis. Thus, the treatment head may laterally be moved into this space, such as may be achieved by a piston / mandrel design. Alternatively, the first and second web support means may be transversely moved along the x-direction of the web. The treatment head may be introduced by a rotational move, or the relative movement of treatment head and web support means may be achieved by combining any of these movements.

For the application of the present invention to articles comprising two parallel hoops connected to a centre piece, a loop forming tool attached to the front face of a treatment head may support appropriate unfolding of the hoops into the correct 3D shape. Whilst such a hoop forming tool may be applied with various treatment head executions, its functioning is explained in further detail in the context of example D.

A further optional feature which is particularly suitable for articles comprising a centre piece material exhibiting a varying width and leg hoops, such as when ears are extending laterally outwardly of a continuous web material. Due to the overlapping of ear material with parts of the leg hoop material or centre piece material upon creation of the 3D shape on the forming head, this part of the material may not be accessible for being appropriately bonded. Thus, a part of these ears may be folded over prior to transferring the web to the web path splitting means. This upstream process step can be performed on a ear flipping drum, which may be combined with various embodiments of the present invention, but is for simplicity described in the context of a specific example (Example E).

The present invention is particularly useful for automated high speed production lines. In the present context, high speed refers to overall web paths speeds of 5 m/sec or more.

### Examples

The present invention is further explained by describing specific process steps and the corresponding equipment set up for particular embodiments. This should, however not be seen in any way limiting, but the skilled person will readily see further variations as well as applications.

### Example-A - Making of disposable articles with overlap side seam

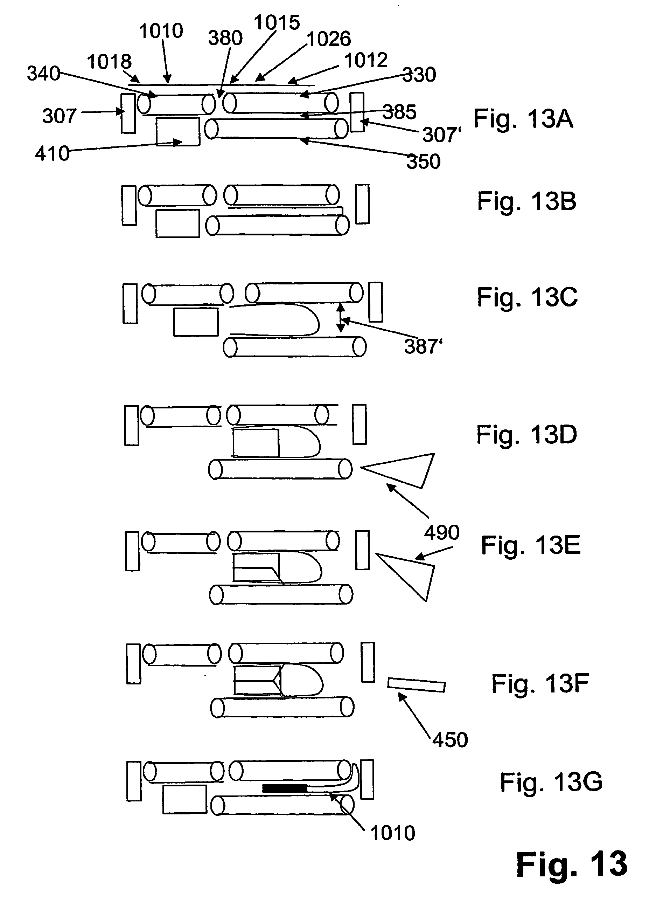

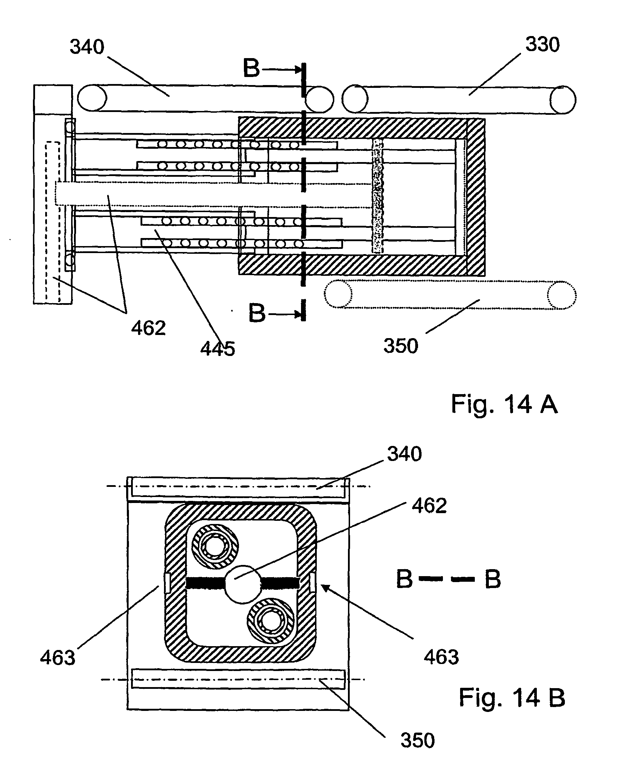

The present invention provides a simple and reliable alternative to produce disposable absorbent articles of the "pant type" with an overlap side seam, such as described in the above, and shown in Fig. 1 C and D. Fig. 8 describes the overall process, and in Fig. 13 A-G the key steps of this example process are depicted. The design principles for a suitable treatment head are shown in Fig 14 A and B, for which elements as described for Fig. 12 are not further explained here.

Typically, several web materials and other materials, such as forming the absorbent core, are added to one web material, in Fig. 8 shown being unreeled from a web supply means 210 to form an essentially endless sequence of semi-finished articles, such as in a web form 1010. This is fed along the predetermined web path 200 at an overall web-path speed 208 via web transfer means 260 and web guide means 270 to a web path splitting means, here shown as a rotating drum, or turret, 300, comprising six essentially identical web treatment sections 301 to 306. On one of these, the web is transferred via guide and/or cutting means 307 (307' belonging to the neighbouring web treatment section) to a first web support means 330, in the present example further supported by a further web support means 340. All web support means are here depicted as vacuum belt systems, which are preferably driven by servo motors with computer controlled speed control. The web may be cut to the appropriate length by a cutting unit (not shown) and a corresponding anvil unit. At the transfer, the first (330) and the optional further (340) web support means are operated at web support speed, which depends on the rotational speed of the rotating drum and the speed of the incoming web, and which is set such that the web essentially continues at the overall web speed 208. A first region (the leading or front region) 1012 of the cut web piece is attached to the first web support means 330, and a second region (the trailing or rear region) 1018 to the further web support means 340. Thus, the web is in contact with the web support means with its second surface 1028, opposing the first surface 1026, which will correspond to the inner surface of the article. Then, the speed of the first and the further web support means 330 and 340 is changed such that both surfaces of the web support means move towards the gap 380 between web support means 330 and 340 with the regions of the web material piece attached. Consequently, the centre region 1015 connecting the front and rear region can bulge and thus - optionally supported by a tucker means, here not shown - move into the gap 380 between the first (330) and the further (340) web support means, where it can be received by the second web support means 350, here shown positioned underneath the first web support means 330 (with underneath referring to the fact, that it is positioned radially inwardly in the rotating drum 300). Thus, the web will move into the gap 385 between the first (330) and the second (350) web support means. The gap width 387, i.e. the distance between the two web support means, is essentially adjusted to accommodate the folded web tightly, but without unduly pressuring it.

When the cut web piece is fully drawn into the gap 385, the gap width 387 is increased, such as by moving the second web support means radially inwardly towards the centre of the drum. Whilst the first (1012) and second (1018) regions remain firmly attached to the respective web support means 330 and 350, the attachment of the centre region 1015 is somewhat loosened to compensate for the increased gap width. Thus, the web piece shows the rough shape of the article (see Fig. 13C), here in the form of a lying "U", with its second surface, which is still attached to the web support means, corresponding to the outer surface of the article, and the opposing surfaces corresponding to the inner surface 1026. The opening between the first and the second region also corresponds to the opening of the article, such as to the waist opening of trousers of a diaper.

When the gap width has reached the predetermined distance 387', a web treatment head 410 is positioned into the opening, such as transversely moving the treatment head, and parts of the web which extend laterally outwardly from the web support means 330 and 350 may be pushed against the treatment head, such as by four guide plates 490, i.e. one for each lateral sides both of the front and rear regions of the web. The guide plates may be arranged movable relative to the web treatment section, or - as indicated in Fig. 13 D and E - may be stationary with regard to the rotating drum. There may be e.g. one guide plate for the left side front ear per treatment section and treatment head, or one may cooperate with consecutive treatment heads.

The guide means such as the guide plates 490 will bend and guide the lateral side portions of the first (1012) and second (1018) region of the web material such that they lie in an overlapping arrangement on the treatment head, which may comprise suitable attachment means 463, such as vacuum suction regions, so as to maintain this relative positioning of the regions. Further, the treatment head may comprise elements to treat the material, such as glue or pressure bonding means, which are appropriately positioned to correspond to the regions of the article, which are to be treated, such as being connected to each other.

Now a treatment tool 450, e.g. an embossing tool, is positioned appropriately relative to the treatment head, and the treatment head and the treatment tool cooperate so as to create the connection. The connecting system may also be re-closable, such as when hook loop or re-usable tape systems are applied.

### Example B - Making of a belted pant article

A further article, which can advantageously be produced by applying the present invention is a "belted pant article" as schematically depicted in Fig. 2A. This article comprises a closed hoop structure 1220 adapted to fit around the waist of a wearer and preferably being elasticized as to fit various sizes and to ease donning, and a centre piece 1210 attached in its front region 1212 and its back region 1218 to the corresponding regions of the hoop structure, such that the centre piece is adapted to fit in the crotch region of the wearer during use.

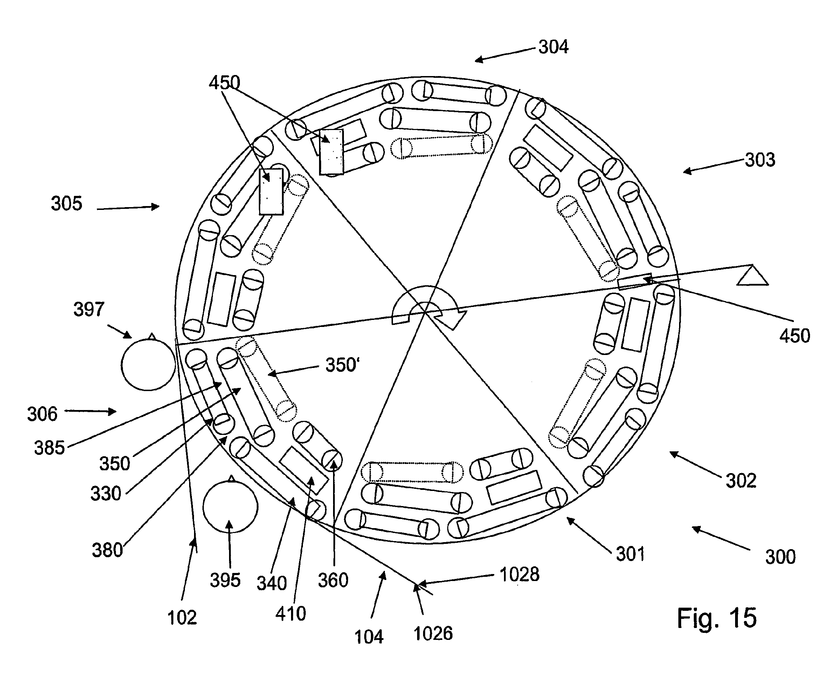

The process steps are schematically depicted in Fig. 15, showing a treatment section similar to the one as shown in Fig. 8 and a suitable treatment head is described in Fig. 12. The starting materials can be two web materials, a first one to form the centre piece and a second one to form the belt or hoop structure. The first web material may be an essentially endless sequence of connected or unconnected absorbent cores, optionally contained between a top sheet and a back sheet, as well known in the art. Typically, though not necessarily, the machine-direction of such cores corresponds to a line from the front to the back through the crotch region of the wearer. The second web may be an elasticized material, preferably a composite so as to accommodate the various requirements of elasticity, strength, user skin friendliness, and so on. This web may also be assembled by arranging various webs side by side, and bond their overlapping lateral side regions together. Such belts may be closed by a permanent seam, or one of the recloseable type, like hook and loop, or tape, or tab and hole based.

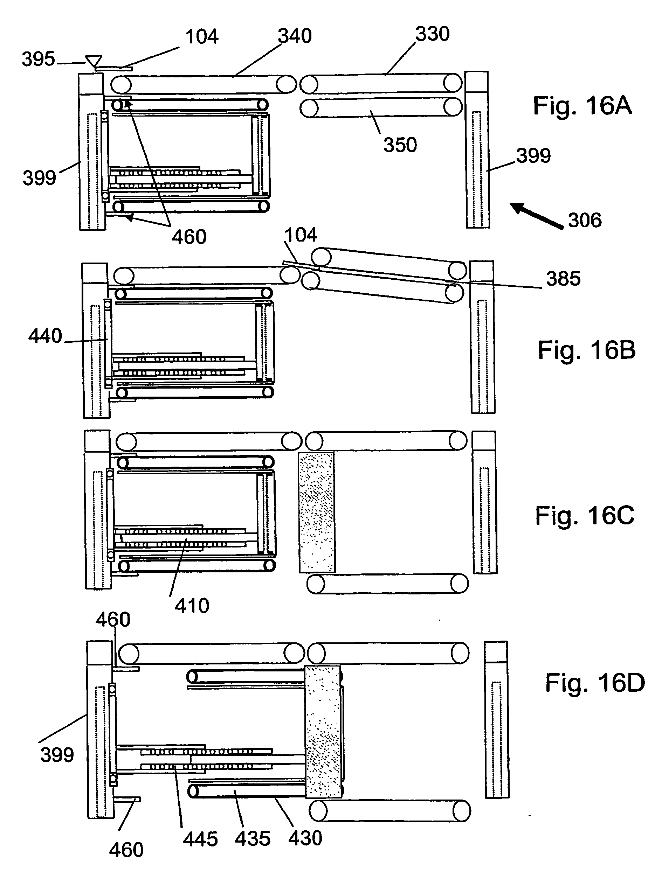

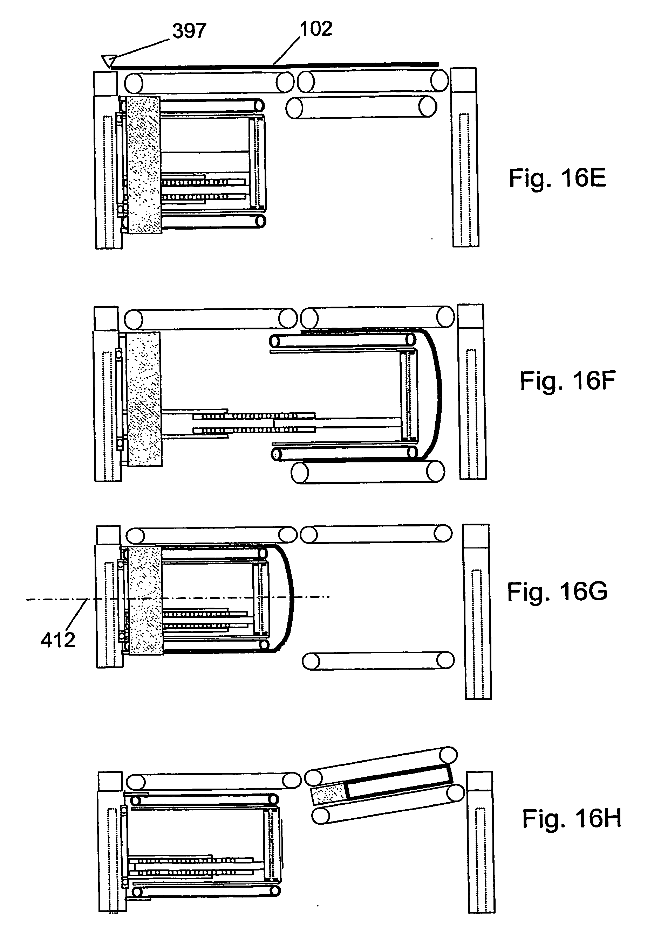

The second material which will form the belt piece 1220 may be delivered as a single layer or a composite web having a web width (y-direction) corresponding to the (non extended) waist circumference. Preferably, it comprises elastic material, and may be extendible in its y-direction, such as by being activated by a set of intermeshing rolls, as described in US-B-5143679. This web is then folded longitudinally, such that the laterally outwardly regions having a width extension of about a quarter of the total width, are folded such that they lay on the centre half region of the web, optionally with a certain overlap so as to ease connecting the outer transversal edges. Optionally, the side edges may already be connected at this point in the process. Alternatively, two webs may be superimposed and their ends bonded together, giving a closed belt with butt type closure on the sides. In yet another embodiment, a wide first web may be folded on both sides such that a gap remains, which is overlaid with a narrow second web. The connection between the first and second web may be by glue, or mechanical fastener, or macro fastener, or heat bonding, or a combination thereof. The thus folded and closed web 104 (see Fig. 15 and 16A) is fed to the rotating drum, here shown to the web treatment section 306, temporarily affixed with its second surface 1028 to web support means 330 and 340 and cut by means of cutting unit 395. First (330) and second (350) web support means may be moveably affixed to the web treatment section frame 399, such as to pivot such as indicated in Fig. 16B so as to allow feeding of the cut belt piece material 104 directly into the gap 385 between the first and the second web support means. Alternatively, the two belts could remain stationary, and the web piece 104 could be transferred by appropriate speed adjustments of the web support means.

As next step, the gap distance 387 of the gap 385 is increased. This gap distance increase may be achieved by pivoting the web support means 350 closer to the centre of the rotating drum, as indicated in Fig. 16C and by the dashed position 350' in Fig. 15. As belt material piece 104 remains affixed to the web support means surfaces at least in the centre region, the laterally outwardly positioned regions will be appropriately released from these surfaces so as to allow opening of the belt structure without unduly extending the belt piece material. If desired, a certain amount of extension may be imparted to these sections.

The treatment head 410 can now be moved into the opened belt structure (see Fig. 16D) by extending the piston system 445 which connects the moveable part of the treatment head at the treatment head base 440 with the web treatment section frame 399. The belt structure can now be transferred onto the surfaces 430 (in Fig. 16D shown as upper and lower) of a transport belt system 435 of the treatment head. The treatment head returns into the original non-extended position, and the belt structure is laterally moved towards the treatment head base 440 by operating of the transfer belt system 435, where it is received by e.g. four waist hoop holding pins 460.

In the meantime, a continuous first web material 102, which may already be prepared so as to form the centre piece 1210 of the article after being cut, is also transferred to the same drum to which the belt piece web material is fed, however angularly offset to the transfer point of web 104, here shown to enter in the region of the neighbouring web treatment section. Thus, the centre piece material 102 may be appropriately cut by a second cutting unit 397, and transferred to web support means 330 and 340. Initially, both web support means 330 and 340 may be positioned levelled and run idle, i.e. the circumferential speed 308 of the turret corresponds to the speed of the incoming web, or they run at equal speed, so as to allow transfer of the web material being fed in slower or faster speed relative to the circumferential speed of the turret in a flat state (see Fig. 16E). The surfaces of the web support means and 340 move towards the gap 380 with regions 1212 and 1218 attached thereto, such that the web material piece 1210 is moved into the gap 380. As a second web support means 350 is in a position close to web support means 330, and is further moving with an appropriate speed, the centre piece will be folded into the gap 385 between web support means 350 and 330, with the front (1212) and rear (1218) regions laying accurately on each other.

As next step, the gap distance 387 of the gap 385 is increased, whilst the front (1012) and rear (1018) regions are held affixed, and centre region 1015 next to the CD fold is somewhat released, so as to compensate for this distance without imparting undue strain in the article. This gap distance increase may be achieved by pivoting the web support means 350 closer to the centre of the rotating drum, as indicated in Fig. 16F and by the dashed position 350' in Fig. 15.

Now, the centre piece can be transferred to the treatment head, here shown by the head moving transversely into the gap, whilst the belt piece material 104 remains "parked" e.g. on the waist hoop holding pins.

Once the treatment head is outside of the gap 385, but before it is fully retracted, it may rotate 90° around its longitudinal axis 412. As the turret is continuing to rotate, it passes by treatment tool 450 (in Fig. 15 shown positioned between sections 302 and 303), which may apply glues to either the facing surface of the belt material or of the centre piece depending on which of the two is positioned outwardly. The treatment head may now retract completely (see Fig. 16G), whilst the waist hoop support pins may have moved apart somewhat so to adjust the positioning and/or tensioning of the belt material, such that the belt material fits appropriately with the centre piece. The treatment head may rotate to its original position and move again into the gap 385, from where the combined belted product may be removed, e.g. by being transferred into a screw feeder running parallel to a tangent of the rotating wheel.

Instead of or in addition to the glue application, the treatment tool 450 may comprise other bonding means, such as for heat, pressure or ultrasonic bonding. Alternatively, the connection can be achieved by releasable adhesive or mechanical closure, or by other connecting means like macro fastener, well known to the person skilled in the art.

### Example C - Making of a 3D article

A particular benefit of the present invention is, that is allows the manufacturing of 3D-articles. A 3D article, as depicted in Fig. 3A and B can be manufactured very efficiently and effectively essentially by the approach very similar to the one as depicted in example A. However, when the connection between the first and second regions are not executed as straight lines / perpendicular to MD/CD , the resulting product will be a 3D article, which presents the key elements constituting it in relaxed and non-deformed state when the product is on the body of the wearer. If desired, the 3D shaped article may be removed from the equipment in the expanded 3D state, or it may be folded, if the creation of creases or crinkles is acceptable.

### Example D - Making of three hoop pants

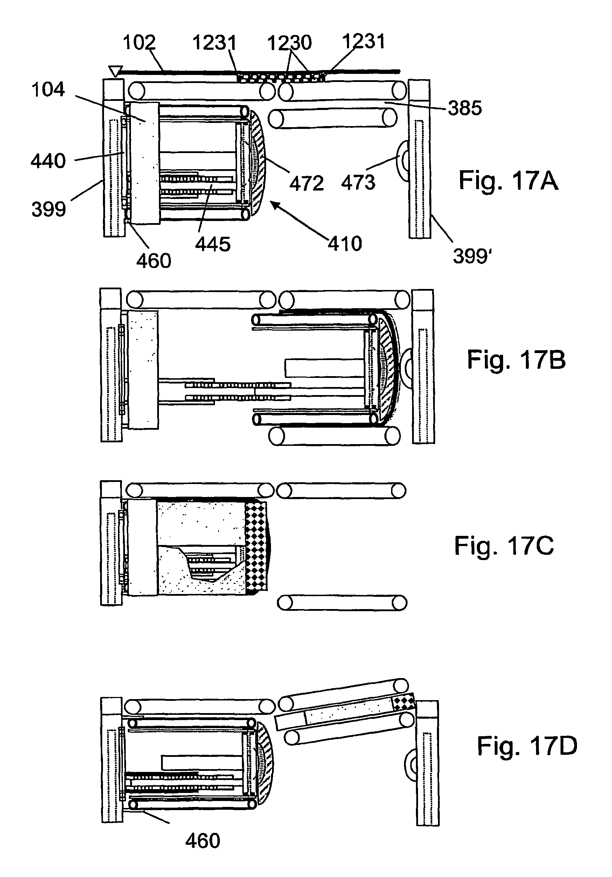

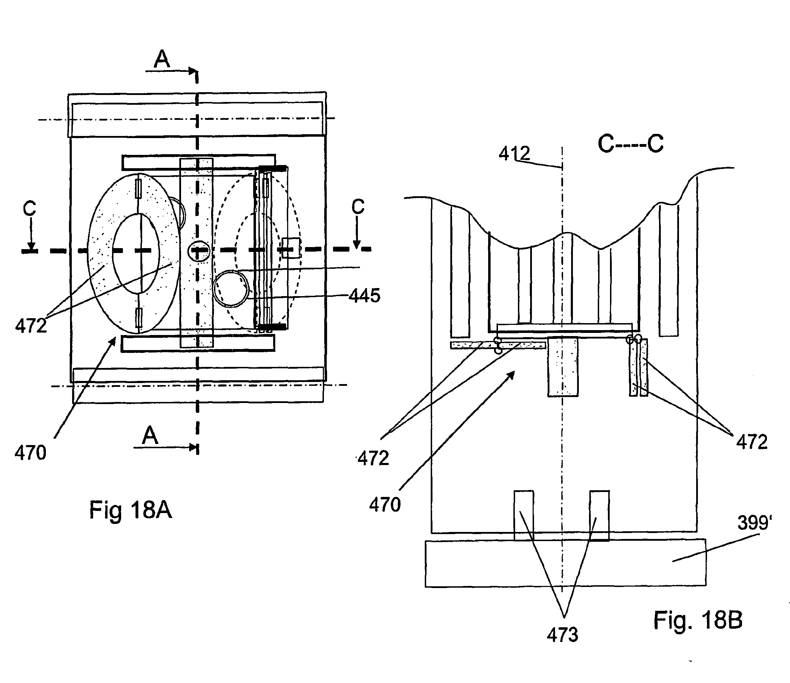

A further application of the present invention may produce articles in the form of pants having three hoop or belt-like structures encircling the waist and the legs, as described hereinabove, and in Fig 2C. These articles comprise a centre piece of material 1210, a waist belt 1220, and two leg belts 1230. The assembling of such an article may be analogous to the assembling of a belted article as described in example B, with the differences explained in the following. The principles of Fig. 15 apply, further details are shown in the process sequence of Fig. 17, and equipment details of the treatment head are shown in Fig. 18.

The first steps of providing the waist hoop material and transferring it onto the waist hoop holding pins are identical to example B, which correspond to Figures 16A to D for example B, thus here omitted. Thus Fig. 17A corresponds to the step shown in Fig. 16E, depicting the waist hoop material 104 on the waist hoop holding pins 460 on the treatment head 410 in its retracted position close to the base 440, which affixes it to the web treatment section frame 399.

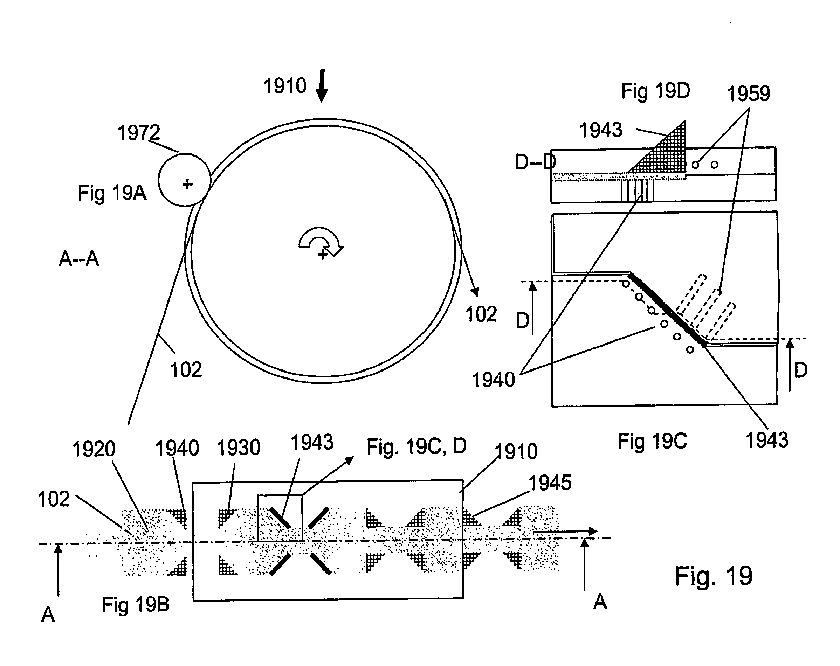

The centre piece 102 is shown in Fig. 17A already cut and positioned on the web support means 330 and 340. Upstream of the rotating drum, the centre piece has been prepared by cutting of leg cut outs, flipping part of the ears, and by being combined with two .layers of leg hoop material 1230. To this end, the leg cut outs 1940 have been prepared by conventional means such as be rotating knives, or laser cutting. As part of the ears would obstruct application of bonding means later in the process, they may be flipped by a simple process step on a flip wheel 1910, as depicted in Fig. 19, showing a cross-sectional (Fig. 19A), a schematic wound off view thereof (Fig. 19B) and an enlarged top (Fig. 19C) and cross-sectional view (Fig. 19D) of a particular section in Fig. 19B. The web 102 is delivered to the wheel via a guide means 1927. The wheel 1910 comprises recessed areas 1920 corresponding to the web regions which are not intended to be flipped over. The other regions 1930 are positioned on elevated shims. Vacuum holes 1940 just aside the shim in the recessed area forces regions 1943 on the shims to turn by 90° into the vertical (see Fig. 19). Corresponding air blast nozzles 1959 positioned in the shims then complete the 180° fold over, such that now a part of the ear 1945is laid flat onto other parts of the ear (see Fig. 19). Subsequently, glue can be applied and the centre piece may be further processed, in this particular case to the combining with the leg hoop pieces.

Also this combination can be performed before it is fed to the rotating drum by a conventional "cut and slip" process, as well known the person skilled in the art. The material for each leg hoop is supplied analogously to the waist hoop material in example B. As shown in fig. 17A, the two layers of one hoop 1230 are connected at their leading and trailing edge to each other, here indicated by bonding region 1231. The hoop materials are positioned in the leg cut out sections of the centre piece, one on each side. They extend laterally outwardly of these, but not further outwardly than the longitudinally extending lateral side margins of the centre piece outside of the leg cut out region. The hoops may be bonded over the entire contacting surface to the centre piece, or over parts of the contacting surface. Preferably, the centre piece is treated in the regions between the leg hoops so as to increase the extensibility thereof. The centre piece is now transferred into the gap 385, and the treatment head 410 moves into this gap.

The treatment head is equipped at its front side with a leg hoop forming device 470, as further schematically depicted in Fig. 18.

The loop forming tool comprises for each leg e.g. two semi-elliptic butterfly baffle rings 472, connected to the front face of the treatment head so as to allow them to lay flat on this front face (as shown in the left portion of Fig. 18A and B) or to stand up vertically from this front face (as shown in the right portion of these figures). Further, the equipment may comprise two projections 473 positioned at the web handling section frame 399' essentially at the point of projection of these baffle rings along the extension of the axis of movement 412 of the treatment head (i.e. opposite of the treatment head base).

When this treatment head is moved into the opening of the web towards web handling section frame 399', guide ploughs 473 fold the side pieces towards the sides of the treatment head (as described in the context of Example A). Because of the attachment of the seal cuff material to the centre pieces, it will open into a hoop structure, which is supported by the loop forming tool, which are "opening" the butterfly wings 472 from the vertical position to an essentially flat position, further supported by the projections.

Then, a T-shaped connecting line on each side of the article can be formed by passing the head by a treatment tool acting from the outside against the article, while the treatment head provides the necessary backpressure from the inside of the article. The so formed T-shaped bonding line connects the lateral sides of the side pieces to each other and to the leg seal cuff. This forming of the connection line is eased by the ear flipping process as described in the above.

The treatment head may now retract towards its base. The centre piece may now be combined with the waist hoop (see fig. 17C), moved by the treatment head into gap 385 again, and the finished article can now be collapsed prior to being discharged.

Prior to collapsing the article for discharge and packing the structure has a true 3D shape (i.e. it cannot be laid flat on a plane with all connecting lines being flat, too). Thus, there may be a certain amount of deformation when the article is removed from the treatment head, and further processed towards packaging. However, when being used, the structure will appropriately open to the body shape adapted 3D form.

### Example E - Method for making an article comprising a leg seal cuff

The method relates to the making of an article comprising leg seal cuffs extending from the crotch area downwardly along the thigh, as shown in Fig. 6. Hitherto, no convenient automated production method for such products is available.

In a first, conventional combining step, which may be executed upstream of the web path splitting means, a first material, which will form the centre piece 1610 of the article, and two further materials, which will from the left and right leg seal cuff, respectively, are provided. In a cut and slip unit, the leg seal cuff materials are cut to their appropriate length, and spaced apart in their x-direction at a distance corresponding to the size of the article (see also Fig. 6D). These pieces are positioned on the first web material, at a position corresponding to the crotch region, and partly overlaying the first material 1610 but extending laterally outwardly of the lateral side margins of the web 1610.

The first web material and the leg seal cuff materials are now connected, such as by a glue line, which may be applied before the webs are combined, or by pressure or ultrasonic bonding. Preferably, the bonding line is curve-linear so as to form the upper edge 1629 of the leg seal cuff. This combined web is now transferred to a web path splitting means, and - analogous to the previous examples - onto a treatment head, which may be of the design as depicted in Fig. 12. Optionally, though not necessarily, the treatment head may comprise the leg hoop forming device, as described in example D.

In a first process option, the treatment head is now moved out of the gap between the web support means, and rotated by 90° around its longitudinal axis 412. In the meantime, two unconnected side panel material layers may already have been fed to the drum and into the gap 385, such that each of these can now be affixed to one of the web support means. The treatment head may now move into the widened gap again, and pick up these two pieces of material on the two opposed surfaces, which are not covered, by the first material. The treatment head may comprise bevelled edges and the respective neighbouring materials may overlap in these bevelled regions, such that the treatment head may rotate appropriately and interact with treatment tools so as to bond the side pieces to the centre piece and the leg seal piece.

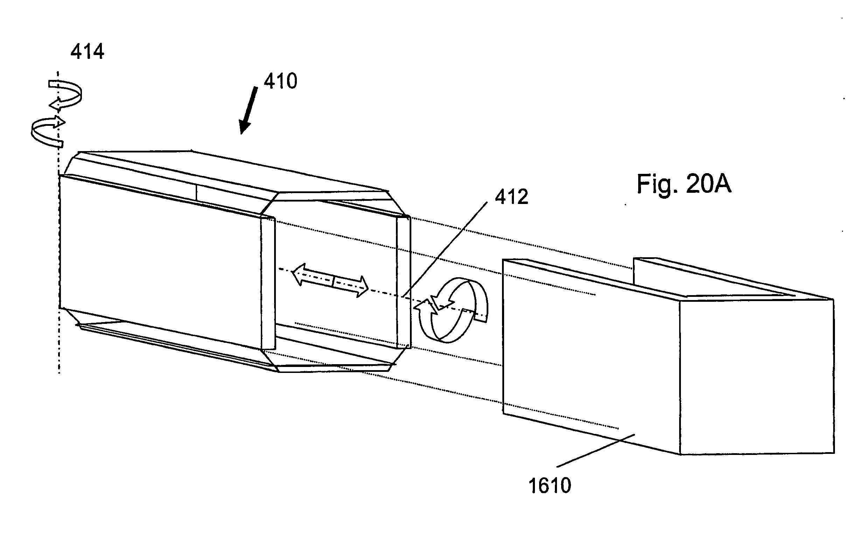

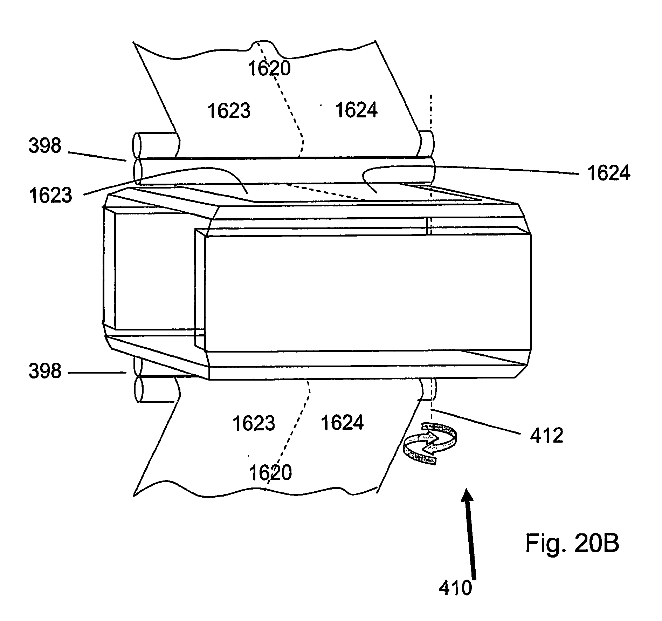

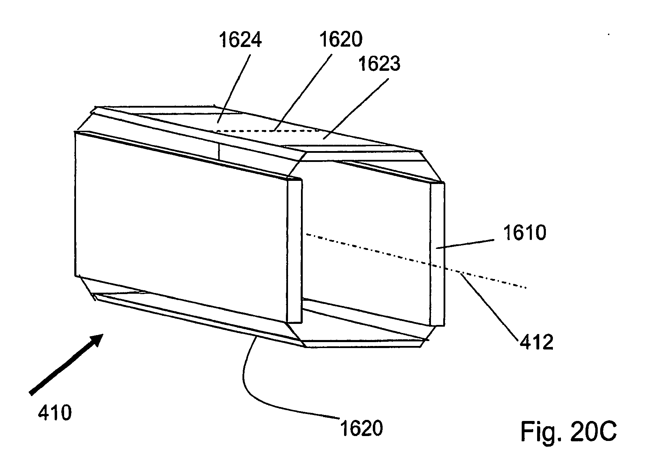

A second process option allows an even more compact design of the overall equipment, as depicted in Fig. 20, showing a schematic perspective view of a treatment head in various positions. To this end, the treatment head 410 can be swivelled out of the plane of the turret (i.e out of the plane of drawing in Fig. 8). When being swivelled in, such as shown if Fig. 20A, the treatment head can also move for and back, and thus move into and out of the gap along its longitudinal axis 412, and thus receive the centre piece 1610 (shown here after being rotated around longitudinal axis 412 and with leg seal cuffs omitted for simplicity). When swivelled out around axis 414, as shown in Fig 20B, the treatment head can receive the two side panel materials 1620 on opposed surfaces (via guide means 398, but with cut and slip and transfer elements not shown for simplicity). Optionally, each side panel may be a composite of two materials 1623 and 1624, which can readily be supplied and manufactured, preferably being pre-bonded at their longitudinally extending side margins. When swivelled back into the turret plane, the machine (or x-) direction of these web pieces 1620 now corresponds to the CD (or y-) direction of the centre piece 1610, as shown in Fig. 20C, thus enabling the use of elastic material extensible in its longitudinal direction as side panel material, which needs extensibility mainly in the product cross direction.

In addition to the equipment design advantages, this process option offers significant freedom for product design variants, with regard to the combination of materials or material properties. For example, the side panel pieces 1624, which corresponds to the waist region in the combined article may have higher or lower elastic contraction forces than the region 1623, or different orientation of the elasticity.

### Example F - Method for making a "hooped" leg seal cuff article

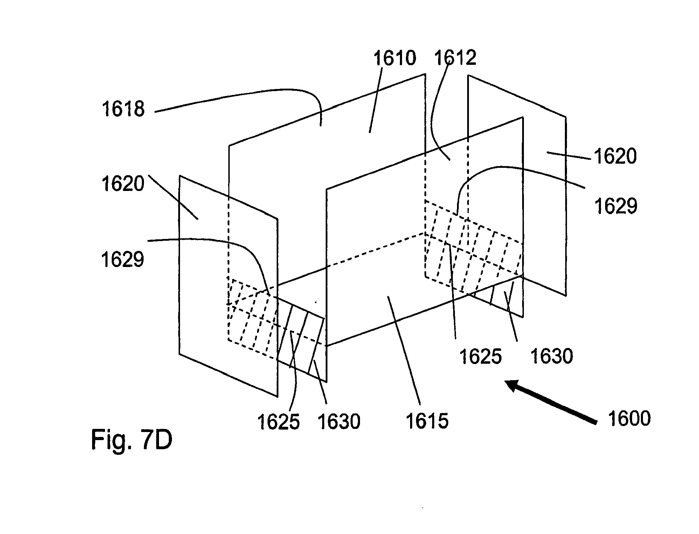

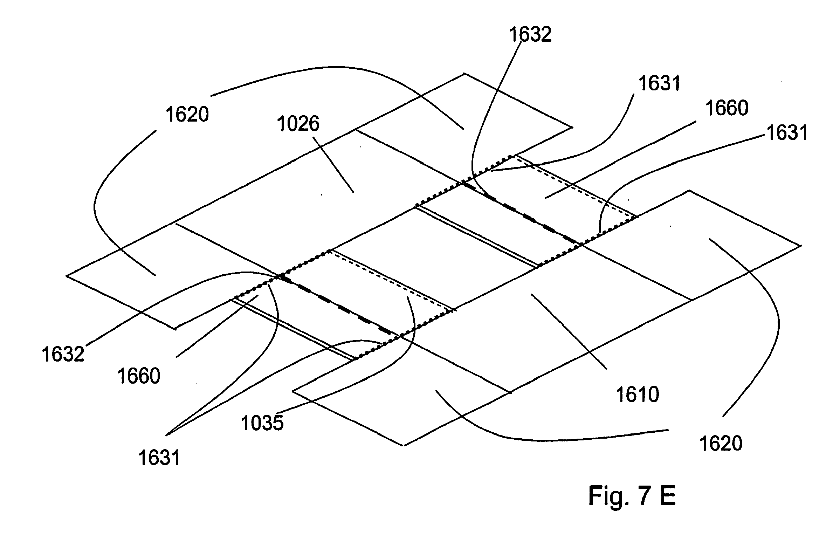

This example relates to the manufacturing of products having a leg seal cuff design combined with leg hoops, as depicted in Fig. 7.

A first web material is provided, which will form the centre piece 1610 of the article, lying on the garment side of the article, i.e. being oriented away from the wearer during use. A second material is provided, which will form the side pieces or side panels 1620. The first and second materials are connected by conventional means, such as a well known "cut and slip" unit, such cut pieces of the second material are attached laterally outwards to the first material so as to form "ear extensions" 1620 as depicted in Fig. 7B. The dimensions of these pieces may be such, that in MD direction 1020 - as long as web 1610 is still a continuous web - one piece of the second material corresponds to twice the side piece length, and that in cross-direction the width corresponds to half the side piece width. Alternatively, the second material may have been cut to the length of the side piece before it is applied. Depending of the type of bonding between the individual pieces selected, the dimensions will be slightly increased to allow for a certain overlap. In an alternative embodiment, a unitary first material may form the side pieces and the centre piece. In this case, a side notch cut would be necessary on both sides of the product, to provide the space required for the leg openings of the formed article.

Two further pairs of a further double layer material are provided which will form the leg seal cuffs 1630 in the article. The double layering may be achieved by providing two layers of material. The double layer materials are cut to a length corresponding to half of the leg encircling circumference, and bonded at their front and trailing edges to each other. Optionally, one material having the cut length corresponding to the fill circumference of the leg may be folded back onto itself, bonded, and cut transversely.

The thusly cut, bonded pieces are spaced apart in their x-direction, at a distance to fit between the spaced apart side pieces. Via conventional guide means, the pieces are positioned on the first web material, at a position corresponding to the crotch region, and partly overlying the first web 1610 but extending laterally outwardly the longitudinally extending lateral side margins of the web 1610. The first web material and the lower layer of the leg seal cuff material (i.e. the one in direct contact with the first web) are now bonded, such as by activating a glue line, which may be applied to the first web prior to the combination. Preferably, this bonding line 1625 has a curve-linear shape. Optionally, the lateral outward margins 1629 of the leg seal cuff materials may be cut curve-linearly.

These two materials are cut and combined by conventional technique, such as by "cut and slip" units, both with their x- or machine direction aligned, and adhesively bonded, so as to create a continuous web formed by the centre piece material 1610 with leg seal cuffs attached thereto. Similar to the process as described for the "belted pant" (example B), the continuous web 1610 with side pieces and double layer leg seal cuff connected thereto is fed to a web path splitting means and into the gap between the two web support means 330 and 350. Upon opening of the gap 385, the front region 1612 of the then cut piece 1610 with the front side regions attached thereto will be attached to the first web support means 330, and the rear region 1618 will be attached to the second web support means 350. The centre region 1615 with the dual layer leg cuff materials attached thereto spans across the gap 385. Thus, at this moment, the web piece 1610 is still an essentially flat structure in a lying open U-form arrangement.

The treatment head corresponds in design to the treatment head 410 as shown in Fig. 17 and 18, comprising the leg hoop forming device 470. The treatment head moves into the U-shaped opening of the centre piece. When this treatment head is moved into the opening of the web, guide ploughs fold the side pieces towards the sides of the treatment head. As the connecting line between the leg seal cuff material and the centre piece is curve-linear, the seal cuff material will open into a hoop structure, which is supported by the loop forming tool, which are "opening" the butterfly wings from the vertical position to an essentially flat position, further supported by the projections 473. Then, a T-shaped connecting line on each side of the article is formed by passing the head by a treatment tool acting from the outside against the article, while the treatment head provides the necessary backpressure from the inside of the article. The so formed T-shaped bonding line connects the lateral sides of the side pieces to each other and to the leg seal cuff. Then, the treatment head moves out of the gap again, and the article is collapsed prior to being discharged. Prior to collapsing the article for discharge and packing, the structure has a true 3D shape (i.e. it cannot be laid flat on a plane with all connecting lines being flat, too). Thus, there may be a certain amount of deformation when the article is removed from the treatment head, and further processed towards packaging. However, when being used, the structure will appropriately open to the body shape adapted 3D form.

### Example G - Making of a diaper with secondary topsheet - Option 1

This example builds on example F and aims at producing an absorbent article, such as a diaper, having a leg hoop design in combination with a secondary topsheet 1712, as schematically shown in Fig. 7 C. This additional web material is cut to the appropriate length, provided with an aperture, and then fed to the web path splitting means analogous to the first web of Example F. The secondary topsheet is connected by CD-oriented connecting lines 1715 to the front and rear regions, and optionally to the side crotch regions of the article on the inwardly, i.e. wearer oriented surface of the article. The leg hoop material may be backfolded prior to the combination with the secondary topsheet to allow application of attachment means, such as adhesive spray, or lines or dots. Alternatively, the secondary topsheet material is designed to be somewhat wider than the width of the centre crotch region, and is not affixed at its longitudinal extending lateral side margins, adjacent to the leg hoop. Upon donning of the article, these wider secondary topsheet regions will fold between the legs of the wearer and the leg hoop, remaining positioned by the wedging effect.

### Example H - Further option for the making of a diaper with leg hoops or leg cuffs

In a further modification of the process as described in examples E and F, the leg hoops or leg cuffs are not connected to the outwardly laying material of the article, but rather onto the wearer oriented ("topsheet" side), refer to Fig. 7D and E, showing the assembly of webs to create such an article. This design may be produced in principle as described hereinabove, except that now the outwardly oriented side portions of the leg hoop material are not folded upwardly to allow opening of the leg opening, but rather downwardly. In this instance, it will be preferable not to have a curve-linear connecting line between the centre and side crotch regions, but rather a straight one.

### Example I - CD-produced articles

Whilst the above examples focused on articles, for which a "main" orientation of the article follows the MD-orientation of the respective centre pieces, the same principles can be applied to a manufacturing process, where the main orientation of the article (e.g. following the line from front to back through the crotch region of the wearer), is aligned with the crotch direction of the web material.

### Example J - Overall diaper production process

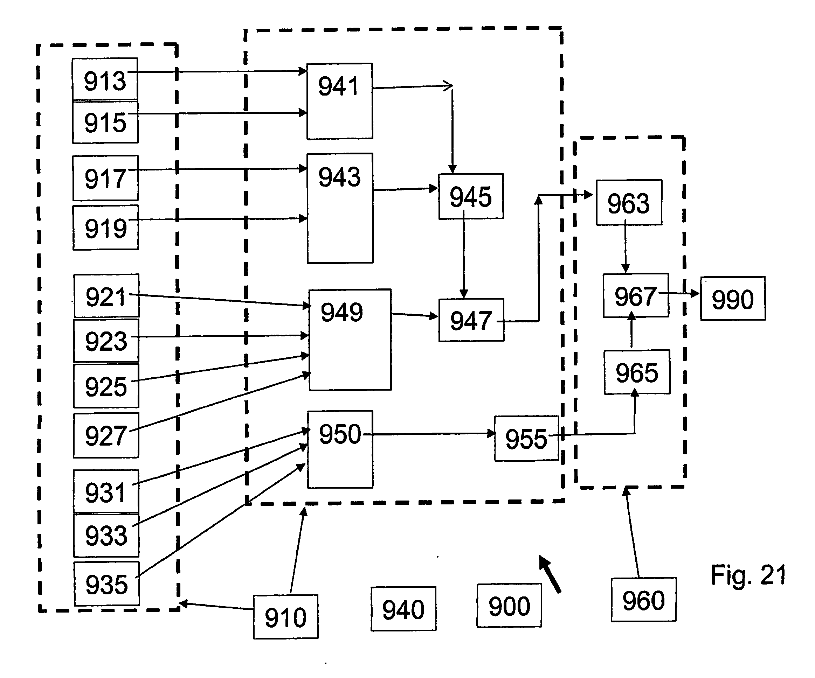

Fig. 21 shows an exemplary process flow chart for the production of a complete articles, here disposable absorbent articles in the form of diapers having a hoop leg seal cuff, as depicted in Fig. 7, and for which the new process steps have been discussed in example E. Both the terminology and the individual conventional process steps will be known to a person skilled in the field of producing disposable absorbent articles, so that such terms are not explained in full detail here. Also details of the individual materials and other processing aids or combining means are well known to a skilled person, and omitted.

The overall process 900 comprises a first group (910) of process steps relating to providing raw materials, a second group (940) of process steps relating to pre-combing raw materials or intermediate structures to intermediate structures, a third group of process steps (960) relating to the combining of the article, and the final process step 990 of packing the article.

In a first group, the leg hoop material 913 and the leg extension / crotch barrier material 915 are provided in roll form, and fed into a first pre-combining step 941 forming the leg hoop and crotch barrier composite. The resulting composite is combined in a conventional "cut and slip" unit 945 with the backsheet material 917, optionally pre-combined in a pre-combining step 943 with a sidepanel material 919.

In a parallel process path the absorbent core and topsheet related items are combined. In a preferred execution, the core is so called "roll-stock" material, i.e. the absorbent materials such as superabsorbent material, and/or fibrous material as well as binding material are forming a continuous web 921, optionally with an envelop material such as a tissue or a non-woven, as may be supplied on a roll or a spool. Alternatively (not shown), the core can be made according to any other core forming process, such as, but not limited to laying down a mixture of e.g. cellulosic fluff and superabsorbent material and glue, and enveloping this in a carrier, such as a tissue or non-woven material.

A further material is the topsheet 923, overlying the article on the wearer side. Typically, this is a non-woven material, supplied on rolls. Many articles comprise barrier leg cuffs, which are relatively narrow strips of non-woven material 925, which may be combined with a thin barrier cuff elastic 927, which are designed to fit into the crotch crease of the wearer during use. Whilst such features are compatible with the present invention, it is believed that the design elements as described herein make barrier cuffs unnecessary, as the sealing and containment performance is already significantly enhanced.

The two intermediate structures leaving the combination station 945 and 949 are further combined in station 947, which still can be conventional web handling.

In a third path, the waist roll stock material 931 and the closure system elements, here shown as a mechanical fastening system comprising hooks 933 and loops 935 are connected in a combining step 950 to the waist belt combination. Optionally, this composite may be folded, as depicted by unit 955.

Thus, the composites leaving the units 947 and 955 may now be transferred to the so called "pant combiner" 960, which may be rotating drum or turret as an example for the web path splitting means, and thus represent typical webs as may be suitably employed in the above described process. Upon appropriate cutting (963 and 965), turning, and opening, and combining (967), the final article may be transferred to the packing unit 990

In contrast to conventional combining steps, the pant combiner is not necessarily the speed limiting factor, as the web path splitting means principle allows the decoupling of the process treatment times from the overall process speed. Henceforth, the present process not only provides automated production capabilities for hitherto not known product designs, but also provides for a multitude of known product designs an efficient production alternative.

## Claims

1. A method for manufacturing shaped articles (1000,1200,1400,1600) from one ore more web materials (100) on a manufacturing equipment, wherein
said articles form a closed hoop structure or a three-dimensional structure and have an inner (1026) and an outer (1028) surface;
said articles being formed by connecting one or more regions of one or more web pieces of one or more web materials (100);
said web materials (100) being essentially flat by having a length dimension (x- direction) and a width dimension (y- direction) exceeding the thickness dimension (z-direction), thus having a first and a second surface along the x-y-direction;
said web materials (100) forming a sequence of web material pieces;
said method being an essentially continuous manufacturing process, comprising at least one web treatment step acting on said inner and outer surfaces simultaneously by a web treatment unit (301,302,...) comprising a web treatment head (410) and a counteracting web treatment tool (450),
said manufacturing equipment comprising a web path splitter means (200) for parallel treatment of at least two web pieces in at least two web treatment sections, each comprising
(i) a treatment head of said web treatment unit (410),
(ii) and at least a first and a second web support means (330,350) for temporarily holding and moving said web material pieeces (100),
said second web support means (350) being positioned z-directionally offset relative to the first web support means (330) thereby forming a gap between said first and second web support means,
wherein the gap distance between said first and second web support means can be varied;
said method comprising steps of
(a) feeding one or more pieces of web material (100) via said web path splitter means (306) to said web treatment sections (301,302,...);
(b) temporarily attaching
(b1) a first region of a piece of a web material to said first web support means, (330)
(b2) and a second region of the same or a different piece of a web material to said second web support means (350);
such that said first and said second regions are positioned in said gap between said first (330) and said second web support means (350);
(c) increasing the z-directional distance of said first and second web support means whilst said first and second regions of said web material(s) remain at least partly affixed to the respective web support means,
so as to increase the gap distance between said web support means and said regions of said web material piece(s) affixed thereto,
(d) positioning said web treatment head (410) in said gap to contact the surfaces of said web material regions oriented towards said web treatment head;
(e) positioning said web treatment head (410) and said web treatment tool (450) relative to each other and to the web material so as to allow them to interact each with one of the opposite surfaces of said web material(s);
(f) treating said first and said second web regions jointly by cooperatively operating said web treatment head (410) and said web treatment tool (450) of said treatment unit, thereby forming the structure of said article;
(g) removing said web treatment head (410) from said gap and/or said article from said head;
(h) optionally further treating said article;
(i) removing said article from said web handling equipment

2. A method according to any of the preceding claims,
wherein said web materials comprise films, textile, woven, or non-woven webs, or composites thereof.

3. A method according to any of the preceding claims,
wherein said first and second regions are comprised in respective different webs; which may be of the same or different material type, orientation, or which may have undergone different pre-treatments.

4. A method according to any of the preceding claims,
wherein said webs material(s) are pre-shaped webs, preferably forming a closed structure like a belt or a hoop.

5. A method according to any of the preceding claims,
further comprising the use of other materials, preferably preassembled pieces, bulk material, or fluids.

6. A method according to any of the preceding claims,
wherein several process steps are executed on one piece of web material

7. A method according to any of the preceding claims,
further comprising any of following treatment steps:
(i) cutting
(ii) pressing
(iii) mechanically activating of webs.

8. A method according to any of the preceding claims,
for the manufacture of shaped articles for being worn on the lower torso, preferably as
(i) disposable garments or
(ii) disposable absorbent articles or
(iii) disposable absorbent pads.

9. A method according to claim 8,
wherein said shaped articles comprise any or all of the following elements
(i) a hoop structure defining the waist opening of the article;
(ii) two hoop structures defining the leg openings of the article;
(iii) a centre piece connecting the front and rear regions through the crotch region of a wearer during use;
(iv) side panel materials positioned in the hip region of the wearer during use;
(v) said centre piece being connected to said leg hoops, preferably by a curved connecting line;
(vi)said side panels being connected to front and back parts of the centre piece, and either to the leg hoop, or to the waist hoop, or to both

10. An apparatus for manufacturing shaped articles from one ore more web materials, said apparatus comprising
(i) a web path splitting means comprising at least two web treatment sections; (ii) a web treatment unit, comprising at least two web treatment elements in the form of a web treatment head and a web treatment tool, which interact at least for the treatment of said web;
(iii) said web treatment sections comprising
(iiia) at least a first and a second web support means comprising moveable surfaces to which said web materials can be temporarily affixed;
(iiib) an element of said web treatment unit, preferably a treatment head;
wherein
said web support means comprise means to freely program the surface speed and direction;
said first and said second web support means are arranged and comprise means to adjust their relative positioning, thereby forming a gap with a variable gap distance between them;
and said web treatment head and said first and second web support means being arranged and comprise means to adjust their relative positioning to allow positioning of said treatment head in said gap.

11. An apparatus according to claim 10,
wherein said web path splitting means is a drum rotatable around its longitudinal axis, preferably comprising two, more preferably four, and most preferably six web treatment sections.

12. An apparatus according to any of claims 10 to 11
comprising further web support means.

13. An apparatus according to any of claims 10 to 12,
wherein said web support means comprise any or all of the following elements:
(i) endless transport belts;
(ii) at least one drive roll;
(iii) a freely programmable drive motor, preferably a servo motor;
(iv) non-contact power and data transfer to said drive roll;
(v) vacuum suction means.

14. An apparatus according to any of claims 10 to 13,
wherein said treatment head comprises any or all of the following elements:
(i) means for affixing web materials;
(ii) means for repositioning the web materials;
(iii) means for repositioning the treatment head;
(iv) rotatably;
(v) translatorily;
(vi) means for expanding or reducing certain dimensions of said treatment head;
(vii) means for treating the web material in cooperation with said treatment tool;
(viii) preferably at least one of the treatment means allowing connecting web material(s);
(ix) a moveable surface, preferably in the form of a transport belt comprising vacuum suction means and a freely programmable drive motor and a non-contact power and data transfer.

15. An apparatus according to any of claims 10 to 14,
wherein a treatment head interacts with one or more treatment tools

16. An apparatus according to any of claims 10 to 15,
wherein a treatment tool interacts with one or more treatment heads.

17. An apparatus according to any of claims 10 to 16,
wherein said treatment tool is affixed
(i) to the web treatment station, and stationary or repositionable relative to said treatment head;
(ii) to the manufacturing apparatus, and thusly consecutively interacting with consecutive treatment heads, as these pass by.

18. An apparatus according to any of claims 10 to 17,
wherein said treatment elements comprise
(i) rolls, or
(ii) anvils, or
(iii) spray or bead application, or
(iv) cutting, or
(v) bonding units.

19. An apparatus according to any of claims 10 to 18,
further comprising sensing devices and control means for the detecting and correcting position of web materials.

20. An apparatus according to any of claims 10 to 19,
for the manufacture of shaped articles for being worn on the lower torso, preferably as
(i) disposable garments, or
(ii) disposable absorbent articles, or
(iii) disposable absorbent pads.

21. An apparatus according to any of claims 10 to 20,
for the manufacture of shaped articles for being worn on the lower torso,
wherein said shaped articles comprise any or all of the following elements
(i) a hoop structure defining the waist opening of the article;
(ii) two hoop structures defining the leg openings of the article;
(iii) a centre piece connecting the front and rear regions through the crotch region of a wearer during use;
(iv) side panel materials positioned in the hip region of the wearer during use;
(v) said centre piece being connected to said leg hoops, preferably by a curved connecting line;
(vi) said side panels being connected to front and back parts of the centre piece, and either to the leg hoop, or to the waist hoop, or to both.

## Patentansprüche

1. Eine Herstellungsmethode zur Herstellung von geformten Artikeln (1000, 1200, 1400, 1600) aus einem oder mehreren Bahnmaterial(ien) (100) auf einer Produktionseinheit, wobei die Artikel ein geschlossenes Bündchen oder eine dreidimensionale Struktur aufweisen und eine innere (1026) und eine äußere (1028) Oberfläche aufweisen;
die Artikel **dadurch** geformt werden, dass eine oder mehrere Region(en) von einem oder mehreren Stück(en) eines oder mehrerer Bahnmaterials/ien (100) verbunden werden;
die Bahnmaterialien (100) im Wesentlichen flach sind, wobei sie eine Längenausdehnung (x-Richtung) und einer Breitenausdehnung (y-Richtung), die beide größer sind als die Dicke (z-Richtung) und die **dadurch** eine erste und eine zweite Oberfläche in der x-y-Orientierung aufweisen;
die Bahnmaterialien (100) eine Reihe von Bahnmaterialstücken bilden;
die Methode ein im Wesentlichen kontinuierlicher Herstellungsprozess ist, in dem wenigstens ein Bahnmaterialbehandlungsschritt vorgenommen wird, in dem mittels einer Bahnmaterialbehandlungseinheit die innere und die äußere Oberfläche simultan bearbeitet wird, wobei die Bahnmaterialbehandlungseinheit einen Bahnmaterialbehandlungskopf (410) und ein gegenwirkendes Bahnbehandlungswerkzeug(450) besitzt;
die Produktionseinheit eine Bahnaufteilung (300) beinhaltet, um wenigstens zwei Bahnmaterialstücke gleichzeitig in wenigstens zwei Bahnmaterialbearbeitungssektionen zu bearbeiten, wobei jede Bahnmaterialbearbeitungssektionen folgendes beinhaltet:
(i) einen Bahnmaterialbehandlungskopf der Bahnmaterialbehandlungseinheit (410)
(ii) und wenigstens eine erste und eine zweite Bahnmaterialauflage (330,350) zum zeitweisen Halten und Bewegen des Bahnmaterials beinhalten;
wobei die zweite Bahnmaterialauflage (350) in der z-Richtung versetzt zu der ersten Bahnmaterialauflage (330) angeordnet ist, wodurch eine Lücke zwischen der ersten und der zweiten Bahnmaterialauflage gebildet wird und der Lückenabstand zwischen der ersten und der zweiten Bahnmaterialauflage variabel eingestellt werden kann;
die Methode folgender Unterschritte enthält:
(a) das Zuführen von einem oder mehreren Stück(en) eines Bahnmaterials (100) über eine Bahnaufteilung (300) zu einer Bahnmaterialbearbeitungssektionen (301,302, ...) ;
(b) dem zeitweisen Befestigen von
(b1) einer ersten Region eines Stückes eines Bahnmaterials an der ersten
(b2) und einer zweiten Region desselben oder einen Teil eines anderen Stückes eines Bahnmaterials an der zweiten Bahnmaterialauflage (350),
Bahnmaterialauflage (330),
so das diese erste und die zweite Regionen in die Lücke zwischen der ersten (330) und der
zweiten (350) Bahnmaterialauflage positioniert werden;
(c) dem Vergrößern des Abstands in der z-Richtung zwischen der ersten und der zweiten Bahnmaterialauflage, während die erste und die zweite Region von diesem oder diesen Bahnmaterial(ien) zumindest teilweise an den entsprechenden Bahnmaterialauflagen fixiert bleiben,
so dass der Lückenabstand zwischen der Bahnmaterialauflage und den daran befestigten Regionen des/r Bahnmaterialstück(en) vergrößert wird;
(d) dem Positionieren des Bahnmaterialbehandlungskopfes (410) in der Lücke, so dass die Oberflächen der Bahnmaterialregionen, die zu dem Bahnmaterialbehandlungskopf ausgerichtet sind, mit diesem in Kontakt treten;
(e) dem Positionieren des Bahnmaterialbehandlungskopfes (410) und des Bahnbehandlungswerkzeugs (450) zueinander und zu den Bahnmaterialien, so dass diese jeweils auf eine unterschiedliche Oberfläche der Bahnmaterialien wirken können;
(f) dem gemeinsamen Bearbeiten der ersten und der zweiten Region des Bahnmaterials durch den Bahnmaterialbehandlungskopf und das Bahnbehandlungswerkzeug der Bahnmaterialbehandlungseinheit, wobei die Struktur des Artikels geformt wird;
(g) dem Entfernen des Bahnmaterialbehandlungskopfes (410) aus der Lücke und / oder des Artikels von diesem Kopf;
(h) dem optionalen weiteren Behandeln des Artikels;
(i) dem Entfernen des Artikels aus der Bahnmaterialbehandlungseinheit.

2. Eine Herstellungsmethode gemäß Anspruch 1, bei der die Bahnmaterialien Folien, textile, gewebte oder nicht-gewebte Materialien oder einen Verbund solcher enthalten.

3. Eine Herstellungsmethode nach einem der vorhergehenden Ansprüche, bei der die ersten und zweiten Regionen in unterschiedlichen Bahnmaterialien enthalten sind, die aus derselben Materialtype bestehen, die dieselbe oder eine andere Orientierung vorweisen, oder die unterschiedlichen Vorbehandlungen unterworfen sein können.

4. Eine Herstellungsmethode nach einem der vorhergehenden Ansprüche, worin die Bahnmaterialien vorgeformte Bahnen sind, die vorzugsweise eine geschlossene Struktur wie einen Gürtel oder ein Bündchen bilden.

5. Eine Herstellungsmethode nach einem der vorhergehenden Ansprüche bei der weiterhin anderen Materialien eingesetzt werden, vorzugsweise vorgefertigte Stücke, Schüttgüter oder Flüssigkeiten.

6. Eine Herstellungsmethode nach einem der vorhergehenden Ansprüche, worin verschiedene Prozessschritte auf ein Bahnmaterialstück angewendet werden.

7. Eine Herstellungsmethode nach einem der vorhergehenden Ansprüche, welche einen oder mehrere der folgenden Bearbeitungsschritte beinhaltet:
(i) Schneiden;
(ii) Drücken;
(iii) mechanische Aktivierung von Bahnmaterialien.

8. Eine Herstellungsmethode nach einem der vorhergehenden Ansprüche zur Herstellung von geformten Artikeln, die im unteren Rumpfbereich getragen werden können, insbesondere:
(i) Einwegbekleidung;
(ii) wegwerfbare absorbierende Artikel;
(iii) wegwerfbare absorbierende Saugkissen.

9. Eine Herstellungsmethode gemäß Anspruch 8, wobei die geformten Artikel eins oder mehrere der folgenden Elemente enthalten:
(i) eine gürtelartige Struktur als Taillenöffnung des Artikels;
(ii) zwei bündchenartige Strukturen als Beinöffnungen des Artikels;
(iii) ein Mittelstück, welches die vorderen und hinteren Bereiche durch den Schrittbereich des Trägers während der Benutzung verbindet;
(iv) Seitenelemente, welche in dem Hüftbereichs eines Trägers während der Benutzung positioniert sind;
(v) eine Verbindung des Mittelstücks mit den bündchenartigen Beinöffnungen, vorzugsweise mittels einer gekrümmten Verbindungslinie;
(vi) eine Verbindung der Seitenelemente zu den vorderen und hinteren Bereichen des Mittelstücks sowie entweder zu den bündchenartigen Beinöffnungen oder der Taillenöffnung oder zu beiden.

10. Eine Vorrichtung zur Herstellung von geformten Artikeln aus einem oder mehreren Bahnmaterialien, die folgendes enthält:
(i) eine Vorrichtung zur Bahnaufteilung mit wenigstens zwei Bahnmaterialbehandlungssektionen;
(ii) eine Bahnmaterialhandlungseinheit mit wenigstens zwei Bahnmaterialbehandlungselementen in Form eines Bahnmaterialbehandlungskopfes und eines Bahnmaterialbehandlungswerkzeugs, welche gemeinsam auf das Bahnmaterial einwirken;
(iii) die Bahnmaterialbehandlungssektionen enthalten
(iiia) wenigstens eine erste und eine zweite Bahnmaterialauflage mit beweglichen Oberflächen, auf die die Bahnmaterialien zeitweise befestigt werden können;
(iiib) einem Element der Bahnmaterialbehandlungseinheit, vorzugsweise einen Bahnmaterialbehandlungskopf;
wobei
die Bahnmaterialauflagen eine Vorrichtung enthalten, durch die die Oberflächengeschwindigkeit und -richtung frei eingestellt werden kann;
die erste und die zweite Bahnmaterialauflage derartig angeordnet sind und Vorrichtungen enthalten, durch die ihre relative Positionierung eingestellt werden kann, wodurch eine Lücke mit einem veränderlichen Lückenabstand eingestellt werden kann;
und der Bahnmaterialbehandlungskopf und die erste und die zweite Bahnmaterialauflage so angeordnet sind und Vorrichtungen enthalten, die relative Positionierung so einzustellen, dass der Bahnmaterialbehandlungskopf in diese Lücke positioniert werden kann.

11. Eine Vorrichtung zur Herstellung von geformten Artikeln gemäß Anspruch 10, wobei die Vorrichtung zur Bahnaufteilung zylindrisch ist und um ihre Längsachse drehbar ist, und welche zwei, in bevorzugter Weise vier, in noch bevorzugterer Weise sechs Bahnmaterialbearbeitungssektionen enthält.

12. Eine Vorrichtung zur Herstellung von geformten Artikeln gemäß Anspruch 10 oder 11, welche weitere Bahnmaterialauflagen enthält.

13. Eine Vorrichtung zur Herstellung von geformten Artikeln nach einem der Ansprüche 10 bis 12, worin die Bahnmaterialauflagen eins oder mehrere der folgenden Elemente enthalten:
(i) Endlostransportbänder;
(ii) wenigstens eine Antriebsrolle;
(iii) einen frei programmierbaren Antriebsmotor, vorzugsweise einen Servo-Motor;
(iv) kontaktfreie Energie- und Datentransmission zu der Antriebsrolle;
(v) Unterdruckversorgungseinheiten.

14. Eine Vorrichtung zur Herstellung von geformten Artikeln nach einem der Ansprüche 10 bis 13, worin der Bahnmaterialbearbeitungskopf eins oder mehrere der folgenden Elemente enthält:
(i) eine Vorrichtung, um die Bahnmaterialien zu befestigen;
(ii) eine Vorrichtung, um die Bahnmaterialien zu repositionieren;
(iii) eine Vorrichtung, um den Bahnmaterialbearbeitungskopf zu positionieren
(iv) durch Rotation oder
(v) durch Translationsbewegung;
(vi) eine Vorrichtung, um bestimmten Maße des Bearbeitungskopfes zu vergrößern oder zu verkleinern;
(vii) eine Vorrichtung um das Bahnmaterial mit dem Bahnmaterialbearbeitungswerkzeug zu bearbeiten;
(viii) wobei vorzugsweise wenigstens eins der Bearbeitungsmittel das Verbinden der Bahnmaterialien zulässt;
(ix) eine bewegliche Oberfläche, vorzugsweise in der Form eines Transportbandes welches eine unterdruckerzeugende Vorrichtung enthält sowie einen frei programmierbaren Antriebsmotor und kontaktfreie Energie- und Daten- Übertragung.

15. Eine Vorrichtung zur Herstellung von geformten Artikeln nach einem der Ansprüche10 bis 14, wobei der Bahnmaterialbearbeitungskopf mit einem oder mehreren Bearbeitungswerkzeugen zusammenarbeitet.

16. Eine Vorrichtung zur Herstellung von geformten Artikeln nach einem der Ansprüche10 bis 15, wobei ein Bahnmaterialbearbeitungswerkzeug mit einem oder mehreren Bahnmaterialbearbeitungsköpfen zusammenarbeitet.

17. Eine Vorrichtung zur Herstellung von geformten Artikeln nach einem der Ansprüche10 bis 16, wobei das Bahnmaterialbearbeitungswerkzeug befestigt ist:
(i) an der Bahnmaterialbearbeitungsstation und stationär oder repositionierbar relativ zu dem Bahnmaterialbearbeitungskopf ist;
(ii) an die Herstellungsvorrichtung und **dadurch** hintereinander mit aufeinanderfolgenden Bahnmaterialbearbeitungsköpfen zusammen arbeitet, wenn diese sich vorbei bewegen.

18. Eine Vorrichtung zur Herstellung von geformten Artikeln nach einem der Ansprüche10 bis 17, wobei de Bearbeitungswerkzeuge eins oder mehrerer der folgenden Elemente enthalten können:
(i) Rollen;
(ii) Ambosse;
(iii) Sprüh- oder Raupenauftragseinheiten;
(iv) Schneidemittel;
(v) Verbindungseinheiten.

19. Eine Vorrichtung zur Herstellung von geformten Artikeln nach einem der Ansprüche10 bis 18, weiterhin enthaltend Detektoren und Kontrollvorrichtungen zum Feststellen und Einstellen der Position der Bahnmaterialien.

20. Eine Vorrichtung zur Herstellung von Artikeln nach einem der Ansprüche 10-19 zur Herstellung von Artikeln welche am unteren Rumpf getragen werden können, vorzugsweise als:
(i) Einwegbekleidung;
(ii) wegwerfbare absorbierende Artikel;
(iii) wegwerfbare absorbierende Saugkissen.

21. Eine Vorrichtung zur Herstellung von Artikeln, welche am unteren Rumpf getragen werden können, nach einem der Ansprüche 10 - 20, wobei die geformten Artikel eins oder mehrere der folgenden Elemente enthalten:
(i) eine gürtelartige Struktur als Taillenöffnung des Artikels;
(ii) zwei bündchenartige Strukturen als Beinöffnungen des Artikels;
(iii) ein Mittelstück, welches die vorderen und hinteren Bereiche durch den Schrittbereich des Trägers während der Benutzung verbindet;
(iv) Seitenelemente, welche in dem Hüftbereichs eines Trägers während der Benutzung positioniert sind;
(v) eine Verbindung des Mittelstücks mit den bündchenartigen Beinöffnungen vorzugsweise mittels einer gekrümmten Verbindungslinie;
(vi) eine Verbindung der Seitenelemente zu den vorderen und hinteren Bereichen des Mittelstücks sowie entweder zu den bündchenartigen Beinöffnungen oder der Taillenöffnung oder zu beiden.

## Revendications

1. Une méthode de fabrication d'articles formés (1000, 1200, 1400, 1600) à partir d'un ou de plusieurs matériaux de nappe (100) sur un équipement de fabrication, dans lequel lesdits articles forment une structure de boucle fermée ou une structure tridimensionnelle et
possèdent une surface interne (1026) et une surface externe (1028) ;
lesdits articles sont formés en connectant une ou plusieurs régions d'un ou de plusieurs morceaux de nappe d'un ou de plusieurs matériaux de nappe (100) ;
lesdits matériaux de nappe (100) sont essentiellement plats, ayant une dimension en longueur (direction x) et une dimension en largeur (direction y) dépassant la dimension en épaisseur (direction z), ayant ainsi une première et une seconde surface le long de la direction x-y ;
lesdits matériaux de nappe (100) forment une séquence de morceaux de matériaux de nappe ; ladite méthode est un processus de fabrication essentiellement continu, comprenant au moins une phase de traitement de la nappe agissant sur lesdites surfaces interne et externe simultanément par une unité de traitement de la nappe comprenant une tête de traitement de la nappe (410) et un outil de traitement réactif de la nappe (450) ;
ledit équipement de fabrication comprend un diviseur de la voie de transport de la nappe (300) pour le traitement parallèle d'au moins deux morceaux de nappe dans au moins deux sections de traitement de la nappe, chacun comprenant
(i) une tête de traitement de ladite unité de traitement de la nappe (410) (ii)et au moins un premier et un second moyen de support de la nappe (330, 350) pour retenir temporairement et déplacer lesdits morceaux de matériau en nappe (100),
ledit second moyen de support de la nappe (350) étant positionné en décalage par rapport au premier moyen de support de la nappe (330) dans la direction z, formant ainsi un écart entre le premier et le second moyen de support de la nappe, la distance de cet écart entre le premier et le second moyen de support de la nappe pouvant être modifié ;
ladite méthode comprenant des phases
(a) d'alimentation d'un ou de plusieurs morceaux de matériaux de nappe (100) via ledit diviseur de la voie de transport de la nappe (300) vers lesdites sections de traitement de la nappe (301, 302, ...);
(b) d'attache temporaire
(b1) d'une première région d'un morceau de matériau de nappe audit premier moyen de support de la nappe (330),
(b2) et d'une seconde région du même ou d'un autre morceau de matériau de nappe audit second moyen de support de la nappe (350);
de manière à ce que lesdites première et seconde régions soient positionnées dans ledit écart entre ledit premier (330) et ledit second (350) moyen de support de la nappe ;
(c) d'accroissement de la distance dans la direction z dudit premier et second moyen de support de la nappe pendant que lesdites première et seconde régions dudit (desdits) matériau(x) de nappe restent au moins partiellement fixées au moyen de support respectif de la nappe, afin d'augmenter la distance d'écart entre ledit moyen de support de la nappe et lesdites régions dudit (desdits) matériau(x) de nappe y étant fixée(s) ;
(d) de positionnement de ladite tête de traitement de la nappe (410) dans ledit écart pour créer un contact entre les surfaces desdites régions des matériaux de nappes orientées vers ladite tête de traitement de la nappe;
(e) de positionnement de ladite tête de traitement de la nappe (410) et dudit outil de traitement de la nappe (450) par rapport l'un à l'autre et par rapport au matériau de nappe de manière à leur permettre d'interagir chacun avec une des surfaces opposées dudit (desdits) matériau(x) de nappe;
(f) de traitement commun desdites première et seconde régions de la nappe en faisant fonctionner coopérativement ladite tête de traitement de la nappe (410) et ledit outil de traitement de la nappe (450) de ladite unité de traitement, formant ainsi la structure dudit article;
(g) de retrait de ladite tête de traitement de la nappe (410) dudit écart et/ou dudit article de ladite tête;
(h) de traitement ultérieur éventuel dudit article;
(i) de retrait dudit article dudit équipement de manipulation de la nappe.

2. Une méthode conforme à revendication 1,
selon laquelle lesdits matériaux de nappe comprennent les films, les textiles, les nappes tissées et non tissées, ou des composés de ces matériaux.

3. Une méthode conforme à l'une des revendications précédentes,
selon laquelle lesdites première et seconde régions sont comprises dans des nappes respectives différentes, qui pourront être un type de matériau et d'orientation identiques ou différents, ou qui pourront avoir subi des prétraitements différents.

4. Une méthode conforme à l'une des revendications précédentes,
selon laquelle ledit (lesdits) matériau(x) de nappe sont des nappes préformées, formant de préférence une structure fermée comme une ceinture ou une boucle.

5. Une méthode conforme à l'une des revendications précédentes,
comprenant également l'utilisation d'autres matériaux, de préférence des pièces pré-assemblées, du matériau en vrac, ou des fluides.

6. Une méthode conforme à l'une des revendications précédentes,
selon laquelle plusieurs étapes du processus sont exécutées sur un seul morceau de matériau de nappe

7. Une méthode conforme à l'une des revendications précédentes,
comprenant également l'une des phases de traitement suivantes :
(i) découpe,
(ii)pressage,
(iii) activation mécanique des nappes.

8. Une méthode conforme à l'une des revendications précédentes,
pour la fabrication d'articles formés conçus pour être portés sur le bas du dos, de préférence sous la forme de
(i) vêtements jetables ou
(ii) articles absorbants jetables ou
(iii) serviettes absorbantes jetables.

9. Une méthode conforme à la revendication 8,
selon laquelle lesdits articles comprennent une ou plusieurs des éléments suivants :
(i) une structure de boucle fermée définissant l'ouverture de l'article pour la taille ;
(ii) deux structures de boucle fermée définissant les ouvertures de l'article pour les jambes ;
(iii) un morceau central connectant les régions antérieure et postérieure par la zone d'entrejambes d'un utilisateur pendant l'utilisation ;
(iv) des matériaux latéraux positionnés dans la région de la hanche de l'utilisateur pendant l'utilisation;
(v) ladite pièce centrale étant connectée auxdites boucles des jambes, de préférence par une ligne courbe de raccordement ;
(vi) lesdits panneaux latéraux étant connectés aux parties antérieure et postérieure de la pièce centrale, et soit à la boucle des jambes, ou à la boucle de la taille, ou aux deux

10. Un appareil pour fabriquer des articles formés à partir d'un ou plusieurs matériaux de nappe, ledit appareil comprenant
(i) un diviseur de la voie de transport de la nappe comprenant au moins deux sections de traitement de la nappe;
(ii) une unité de traitement de la nappe, comprenant au moins deux éléments de traitement de la nappe sous la forme d'une tête de traitement de la nappe et d'un outil de traitement de la nappe, interagissant au moins pour le traitement de ladite nappe ;
(iii) lesdites sections de traitement de la nappe comprenant
(iiia) au moins un premier et un second moyen de support de la nappe comprenant des surfaces mobiles auxquelles lesdits matériaux de nappe peuvent être temporairement fixés ;
(iiib) un élément de ladite unité de traitement de la nappe, de préférence une tête de traitement ;
dans lequel
ledit moyen de support de la nappe comprend des moyens de programmer librement la direction et vitesse de la surface ;
lesdits premier et second moyens de support de la nappe sont arrangés et comprennent les moyens d'ajuster leur positionnement relatif, formant ainsi un écart avec une distance d'écart variable entre eux ;
et ladite tête de traitement de la nappe et lesdits premier et second moyens de support de la nappe étant arrangés et comprenant les moyens d'ajuster leur positionnement relatif afin de permettre le positionnement de ladite tête de traitement dans ledit écart.

11. Un appareil conforme à la revendication 10,
dans lequel ledit diviseur de la voie de transport de la nappe est un tambour pouvant réaliser une rotation sur son axe longitudinal,
de préférence comprenant deux, ou mieux quatre, ou bien mieux six sections de traitement de la nappe.

12. Un appareil conforme à l'une des revendications 10 à 12
comprenant d'autres moyens de support de la nappe.

13. Un appareil conforme à l'une des revendications 10 à 12,
dans lequel lesdits moyens de support de la nappe comprennent un ou plusieurs des éléments suivants :
(i) des courroies de transport sans fin ;
(ii) au moins un rouleau d'entraînement ;
(iii) un moteur d'entraînement librement programmable, de préférence un servomoteur ;
(iv) une alimentation et un transfert de données sans contact audit rouleau d'entraînement;
(v) moyens d'aspiration à vide.

14. Un appareil conforme à l'une des revendications 10 à 13,
dans lequel ladite tête de traitement comprend un ou la totalité des éléments suivants :
(i) des moyens de fixer des matériaux de nappe ;
(ii) des moyens de repositionner les matériaux de nappe ;
(iii) des moyens de repositionner la tête de traitement
(iv) par rotation ou
(v) par translation ;
(vi) des moyens d'étendre ou de réduire certaines dimensions de ladite tête de traitement;
(vii) des moyens de traiter le matériau de nappe en coopération avec ledit outil de traitement;
(viii) de préférence au moins un des moyens de traitement permettant de connecter le(s) matériau(x) de nappe ;
(ix) une surface déplaçable, de préférence sous la forme d'une courroie de transport comprenant des moyens d'aspiration à vide et un moteur d'entraînement librement programmable et une alimentation et un transfert de données sans contact.

15. Un appareil conforme à l'une des revendications 10 à 14,
dans lequel une tête de traitement interagit avec un ou plusieurs outils de traitement.

16. Un appareil conforme à l'une des revendications 10 à 15,
dans lequel un outil de traitement interagit avec une ou plusieurs têtes de traitement.

17. Un appareil conforme à l'une des revendications 10 à 16, dans lequel ledit outil de traitement est fixé
(i) au poste de traitement de la nappe, et stationnaire ou repositionnable relativement à ladite tête de traitement;
(ii)à l'appareil de fabrication, et interagissant ainsi consécutivement avec les têtes de traitement consécutives, lorsque celles-ci défilent.

18. Un appareil conforme à l'une des revendications 10 à 17,
dans lequel lesdits éléments de traitement comprennent
(i) des rouleaux, ou
(ii) des enclumes, ou
(iii) application d'une pulvérisation ou nervure, ou
(iv) une découpe, ou
(v) unités de fixation.

19. Un appareil conforme à l'une des revendications 10 à 18,
comprenant également des capteurs et moyens de contrôle pour détecter et corriger la position des matériaux de nappe.

20. Un appareil conforme à l'une des revendications 10 à 19,
pour la fabrication d'articles formés conçus pour être portés sur le bas du dos, de préférence sous la forme de
(i) vêtements jetables, ou
(ii) articles absorbants jetables, ou
(iii) serviettes absorbantes jetables.

21. Un appareil conforme à l'une des revendications 10 à 20,
pour la fabrication d'articles formés conçus pour être portés sur le bas du dos, selon laquelle lesdits articles formés comprennent un ou plusieurs éléments suivants
(i) une structure de boucle définissant l'ouverture de l'article pour la taille ;
(ii) deux structures de boucle définissant les ouvertures de l'article pour les jambes ;
(iii) un morceau central connectant les régions antérieure et postérieure par la zone d'entrejambes d'un utilisateur pendant l'utilisation ;
(iv) des matériaux latéraux positionnés dans la région de la hanche de l'utilisateur pendant l'utilisation ;
(v) ladite pièce centrale étant connectée auxdites boucles des jambes, de préférence par une ligne courbe de raccordement ;
(vi) lesdits panneaux latéraux étant connectés aux parties antérieure et postérieure de la pièce centrale, et soit à la boucle des jambes, ou à la boucle de la taille, ou aux deux.
